# EUROPEAN PATENT APPLICATION

(11) **EP 2 033 598 A1**
(43) Date of publication of application: **11.03.2009**
(21) Application number: 07022543.8
(22) Date of filing: 21.11.2007
(51) Int. Cl.: A61F 2/28, A61L 27/42, A61L 27/54, C07K 14/495

(54) **Calcium phosphate based delivery of growth and differentiation factors to compromised bone**

(30) Priority: 05.09.2007 US 970258 P; 30.10.2007 US 928889
(71) Applicant: DePuy-Biotech Gmbh, 07607 Eisenberg (DE)
(72) Inventor: Venbrocks, Rudolf A., 07607 Hainspitz (DE); Kinne, Raimund W., 07743 Jena (DE); Jandt, Klaus D., 07751 Jena (DE); Bossert, Jörg, 07616 Bürgel (DE); Hortschansky, Peter, 07749 Jena (DE); Schmuck, Klaus D., 07552 Gera (DE)
(74) Representative: Jany, Peter

(57) **Abstract**

Resorbable calciumphosphate (CaP) based compositions comprising growth and differentiation factors (GDF) and their uses in bone regeneration and preventive treatment, in particular of osteoporotic bone, are disclosed.

## Description

### Field of the Invention

This invention relates to compositions comprising growth and differentiation factor(s) (GDF(s)) and calcium phosphate (CaP) material as a carrier ("CaP-GDF"). The invention, in particular, relates to biocompatible, osteoinductive and/or osteogenetic compositions for use in, e.g., bone regeneration, osseous augmentation, fracture prevention, tissue repair, and reinforcement in bone fractures, bone implants, and prostheses.

### Background of the Invention

The publications and other materials, including patents and accession numbers, used herein to illustrate the invention and, in particular, to provide additional details respecting the practice, are incorporated herein by reference.

The repair, regeneration, and augmentation of bone tissue in fractures have been subject to research for many years. With the increase of osteoporosis and other bone weaknesses in aging populations, the demands on bone regeneration technology, in particular implant technology, have heightened. In an osteoporotic patient, the bone mass may be reduced considerably. When the reduction is at about 40%, the facture threshold has been reached. About 50% of such patients obtain fractures due to regular everyday activities, with fractures in the femoral neck and the spine being particularly common. In those patients, the natural bone regenerative processes are compromised, thus resulting in poor healing of fractures. Also implants that might work well in young adults, might not work well in such patients. Due to the relative stiffness of many available implants, in patients with compromised regenerative processes, the surrounding bone might, after implantation, be exposed to stresses from the implant that cause follow-up fractures (Baroud and Bohner, Joint Bone Spine 73, 144-150 (2006). Currently 67% of all osteoporotic patients with the most common type of vertebral body implants suffer from such a follow-up fracture within one year.

Several members of the transforming growth factor β (TGF-β)/bone morphogenetic protein (BMP) superfamily, in particular, BMP-2 and BMP-7, have been employed in orthopedic and trauma surgery to accelerate and enhance bone regeneration in individuals with fractures, so as to improve fracture healing or to initiate spinal fusion. However, the use of these factors also bears promise in the treatment of individuals with compromised bone metabolism, such as osteoporotic or elderly patients. Soluble BMP released from rapidly degrading carriers, such as collagen, have been favored. For example, human recombinant BMP-2 produced in mammalian cell lines with collagen from cattle as its carrier is marketed under the name InFuse® (Medtronic Sofamor Darnek, USA) and InductOs® (Wyeth, USA). Commercially available BMP-7, which is marketed under the name Osigraft® (Stryker Biotech, USA), is also bound to collagen from cattle.

Next to growth-enhancing proteins such as BMPs, other avenues have been pursued in reconstructive surgery to replace damaged tissue. Thus, cartilage regeneration systems in which isolated chondrocytes are injected into a damaged area in the context of a polymer scaffold have been used (see, e.g., Atala et al., J. Urol. 150:747 (1993); Freed et al., J. Cell. Biochem. 51:257 (1993) and references cited therein). Similar seeded scaffold systems have been studied in the context of bone repair. Here, osteoblasts are utilized in conjunction with polymeric or ceramic supports (see, e.g., Elgendy et al., Biomat. 14:263 (1993); lshaug et al., J. Biomed. Mater. Res. 28:1445 (1994)).

Polymethylmethacrylate (PMMA) is a bone cement widely used in orthopedics to remodel lost or damaged bone. As a synthetic polymer, it is supplied in a powder with liquid methylmethacrylate (MMA). When mixed, a dough-like cement results that gradually hardens. While PMMA is biologically compatible, it has been considered a possible carcinogen and has been linked to cardiopulmonary events due to hypertension. It has a Young's modulus (elasticity modulus) between callous bone and cortical bone, namely about 2000 MPa, and is not resorbed upon bone-remodeling.

Calcium phosphate ceramics have high biocompatibility and exhibit good to excellent affinity for protein reagents (see, e.g., Ijntema et al., Int. J. Pharm. 112:215 (1994); Itokazu et al., J. Orth. Res. 10:440 (1992); Shinto et al., J. Bone Joint Surg. 74-B:600 (1992); and Uchida et al., J. Orth. Res. 10:440 (1992)). Most of these calcium phosphate materials consist of prefabricated, sintered hydroxyapatite in either granule or block forms. However, these preparations often have properties that impair their effective use in the repair of skeletal defects. Granulate may not stick together and may otherwise be structurally weak. Blocks, such as hydroxyapatite blocks, may be hard to model, which render them difficult to use in implantation, which requires the formation of shapes determined by the individual defect.

In general, all of these products are ceramics, produced by high temperature sintering and are often biologically non-resorbable. Other porous, non-resorbable materials are based on coral material and are marketed under the name HA RESORB and HAPSET in the dental market.

US. Pat. Nos. Re. 33,221 and Re. 33,161 to Brown and Chow teach preparation of calcium phosphate remineralization compositions and a finely crystalline, non-ceramic, gradually resorbable hydroxyapatite (HA) material based on calcium phosphate compositions, which are precursors of hydroxyapatite (HA) and are biologically compatible. These compositions have, in contrast to many other calcium phosphate biomaterials, two advantageous properties: (1) self-hardening to form a mass with sufficient strength for many medical and dental applications, and (2) when implanted in bone, the material resorbs slowly and is completely replaced by new bone formation with no loss in the volume or integrity of the tissue that receives the implant.

Hydroxyapatite cements were shown to have desirable biomechanical characteristics in vertebral body structures reinforced with such cements (Seino et al., J Orthop. Sci. 8:50-54 (2003).

A similar calcium phosphate system based on tetracalcium phosphate (TTCP) and monocalcium phosphate (MCP) or its monohydrate form (MCPM) and which forms hydroxyapatite (HA) is disclosed by Constantz et al. (U.S. Pat. Nos. 5,053,212 and 5,129,905).

U.S. Pat. Nos. 5,650,176; 5,676,976; 5,683,461; 6,027,742 and 6,117,456 to Lee et al., describe a type of calcium phosphate composition that comprises an amorphous calcium phosphate as a reactant, a promoter and an aqueous liquid to form a hardening paste. This system provides a bioactive ceramic material that is biocompatible, bioresorbable and workable for long periods of time at room temperature. The bioactive ceramic material may be formed at low temperatures, is readily formable and/or injectable and yet can harden to high strength upon further reaction. The bioactive ceramic material contains so called poorly crystalline apatitic calcium phosphate solids with calcium-to-phosphate (Ca/P) ratios comparable to naturally occurring bone minerals and has stiffness and fracture roughness similar to natural bone.

US Pat. Appl. No. 20020187104 to Li et al., describes calcium phosphate delivery vehicles for osteoinductive proteins, such as BMP-2 and BMP-7. An everesent agent is designed to prevent the formation of an unitary monolithic structure. Li's biocompatible and bioresorbable calcium phosphate material self-granulates and disperses into hardened macroparticles subsequent to administration. The particles are designed to be sufficiently large to prevent an inflammation reaction. The osteoinductive proteins which may be dispersed homogenously throughout the volume of the individual particle are designed to stimulate osteogenic activity *in vivo.*

The bone morphogenic factors, BMP-2 and BMP-7, are currently being used in clinical practice in combination with collagen from cattle. However, BMP-2 and BMP-7 are associated with side-effects such as the induction of tumors of haemapoietic cells, ectopic ossification, and systemic antibody formation (Harwood et al., Expert Opin Drug Saf 4(1): 75-89 (2005)). GDF-5 defines a distinct structural and functional subgroup of BMPs and shares 40-50% protein-sequence identity (38% sequence identity on the mRNA level) with BMP-2 and BMP-7 (osteogenic protein-1, or OP-1). GDF-5 is expressed in the prechondrogenic mesenchyme and can induce/promote the formation of the initial condensations in micromass cultures, while e.g., the widely used BMP-2, BMP-3 and activin act at the later steps of chondrogenesis (P. H. Francis-West, Development 126, 1305-1315 (1999)). GDF-3's sequence identity on the mRNA level to BMP-2 is, with 20%, even lower. While other members of the BMP family are expressed at different embryonic stages and in different regions of developing bone, GDF-3 is expressed in the osteogenic zone of newly calcifying bone (Jones Mol. Endo 6(11), 1961-68 (1992)).

Experiments with GDF-5 provided, in comparison to BMP-2, varying results, but showed that certain collagen matrixes appeared to influence the activity of GDF-5 (Spiro, Biochem Soc. 28(4) 362-368 (2000); Spiro, Anat. Record 263, 388-395 (2001)). Purified human GDF-5 showed bone-forming potential when reconstituted with allogenic inactivated bone matrix (Hoetten, Growth Factors 13, 65-74 (1996)). When used with rat bone residue, recombinant GDF-5 was considerably less active than BMP-7 (OP-1). It was also reported that GDF-5 when used with hydroxyapatite (HA) showed by itself very little osteogenic activity, but that it displayed, when administered in conjunction with culture-expanded bone marrow mesenchymal stem cells (MSC), a considerable osteogenic response (J. Biomed Mater Res A 68(1), 168-76 (2004)).

Thus, there is a need for a composition that comprises GDF or a functional fragment thereof, and a vehicle conducive for unfolding GDF's osteogenic potential. The composition should, in a preferred embodiment, contain carrier material that is biocompatible and readily resorbable. GDFs should preferably be released at a rate conductive for osteogenesis. Such a composition is preferably injectable, promotes homogeneous distribution of GDF throughout the resorbable material, and thus permits controlled- release of the active substance. And finally, the composition should form a material displaying an advantageous resorption rate that is adjusted to osteogenesis, thus facilitating cell migration and infiltration for secretion of extracellular bone matrix as well as vascularisation.

Advantageously, a high surface area may be provided for enhanced resorption and release of active substance, as well as increased cell-matrix interaction.

### Brief Description of the Figures

Fig. 1 shows in (A) a nucleotide sequence encoding the MPB-GDF-5 fusion protein shown in (B), which will, subsequent to cleavage, result in the mature GDF-5 protein shown in (C).
Fig. 2 shows the relative increase of bone density in lumbar vertebral bodies upon administration of hydroxyapatite (HA), either alone or in combination with rhBMP-2 and rhGDF-5 ("rhBMP").
Fig. 3 depicts the results of compression strength measurements upon administration of hydroxyapatite (HA), either alone or in combination with rhBMP-2 at an optimal dosage after 3 months.
Fig. 4 depicts the results of compression strength measurements upon administration of hydroxyapatite (HA), either alone or in combination with rhGDF-5 at a minimal dosage after 9 months.
Fig. 5 shows the relative increase of bone volume in lumbar vertebral bodies upon administration of hydroxyapatite (HA), either alone or in combination with rhBMP-2, both at the injection channel (a) and at a remote area (here at a distance of about 10 mm) (b).
Fig. 6 shows the relative increase of bone volume in lumbar vertebral bodies upon administration of hydroxyapatite (HA), either alone or in combination with rhGDF-5, both at the injection channel (a) and at a remote area (here at a distance of about 10 mm) (b).
Fig. 7 shows the increase of the diameter of bone trabeculae [µm] in lumbar vertebral bodies upon administration of hydroxyapatite (HA), either alone or in combination with rhBMP-2, both at the injection channel (a) and at a remote area (here at a distance of about 10 mm) (b).
Fig. 8 shows the increase of the diameter of bone trabeculae [µm] in lumbar vertebral bodies upon administration of hydroxyapatite (HA), either alone or in combination with rhGDF-5, both at the injection channel (a) and at a remote area (here at a distance of about 10 mm) (b).
Fig. 9 shows the relative increase in osteoblast surface [%] in lumbar vertebral bodies upon administration of hydroxyapatite (HA), either alone or in combination with rhBMP-2, both at the injection channel (a) and at a remote area (here at a distance of about 10 mm) (b).
Fig. 10 shows the relative increase in osteoblast surface [%] in lumbar vertebral bodies upon administration of hydroxyapatite (HA), either alone or in combination with rhGDF-5, both at the injection channel (a) and at a remote area (here at a distance of about 10 mm) (b).

### Summary of the Invention

The present invention is directed at a composition for delivering recombinant growth and differentiation factor(s) comprising a bioresorbable calcium phosphate material; and a recombinant GDF, in particular GDF-5 and/or GDF-3, as a biologically active agent. The composition is preferably cell-free and/or preferably injectable. The recombinant GDF-5 is, in one embodiment of the invention, a non-glycosylated human recombinant GDF-5. The composition may comprise at least one further osteogenic and/or osteoinductive protein selected from bone morphogenic proteins and/or further GDFs and/or a transforming growth factors. The recombinant GDF-5 may comprise SEQ ID NO: 1: MNSMDPEST.

The calcium phosphate material may be a calcium phosphate such as, but not limited to, monocalciumphosphate anhydrite, calciumhydrogenphosphate dihydrate, calciumhydrogenphosphate anhydrite, hydroxyapatite, a-tricalcium phosphate, β-tricalcium phosphate, fluorapatite, or a combination thereof. The calcium phosphate material may, upon hardening, be strongly bioresorbable. Also, the calcium phosphate material may, upon hardening, comprise interconnecting pores. The composition may further comprise a bioerodible polymeric structure such as a polysaccharide, peptide, protein, synthetic polymer, natural polymer, a synthetic or natural copolymer, or combinations thereof. The polymeric structure may be a microscaffold(s) having a porous structure. Such microscaffold(s) may be between 200 µm and 2 mm, preferably 500 µm and 1 mm in size and/or may have a pore channel diameters of about 30 to 500 µm, preferably of about 100 to 200 µm. The microscaffold(s) preferably have a size that is less than the size of the inner diameter of the syringe used for injection. However, the microscaffold(s) may have diameters that exceed the inner diameter of the syringe used for injection. The polymeric structure/microscaffold may favorably be made from chitosan and/or a poly-lactic-acid/ poly-e-caprolactone (PLA-PCL) copolymer but may also be made of any other degradable polymer. It may further be coated with a biocompatible nanocrystalline calcium phosphate such as, but not limited to, monocalciumphosphate anhydrite, calciumhydrogenphosphate dihydrate, calciumhydrogenphosphate anhydrite, hydroxyapatite, a-tricalcium phosphate, ß-tricalcium phosphate, fluorapatite, or a combination thereof.
The calcium phosphate material may, upon hardening, be strongly bioresorbable and/or comprises phases that resorb at different rates, e.g., a first phase that resorbs at first rate and which may comprise pores having an average diameter of less than 1 µm and which may be in particles the first phase essentially consist of or comprises, and a second phase that resorbs at a second rate which exceeds that of said first rate. Any pores that may be part of the first phase may preferably include the second phase.

The compostion may, in a certain embodiment, comprise a gas-forming agent such as, but not limited to, hydrogen, carbon dioxide, air, nitrogen, helium, oxygen, or argon.

The composition may, upon hardening, have a compression strength of more than 4 MPa such as between about 5 MPa and about 200 MPa, preferably of more than about 10 MPa, more than about 20 MPa, more than about 30 MPa, preferably between about 20 MPa and about 200 MPa, between about 20 MPa and 100 MPa or between 20 and 40 MPa.

The invention is also directed at a method for furthering bone formation comprising:
administering to a bone location, such as a fractured and/or osteoporotic bone of a mammal, the above composition in a bone-forming effective amount. The composition may be administered in one or more doses of less than 100 µg. Said composition, upon administration, may result in an increase of at least about 20%, 25%, 30%, 35%, or 40% in bone volume at said bone location. This method may also include measuring the bone mineral density (BMD) of the respective bone and determining a lower threshold value of said BMD.

The invention is also directed at a kit comprising:
(a) the bioresorbable calcium phosphate material as described herein in one container;
(b) the above biologically active agent in the same or a separate container; and
(c) in a further container, instructions of how to use (a) and (b).

The biologically active agent may be admixed in a buffer solution or may be admixed with the bioresorbable calcium phosphate material. The kit may further comprise tools for kyphoplasty, vertebroplasty or a porous pouch. The pouch can also comprise, consist of, or essentially consist of a resorbable material, for example, Chitosan, which can, for example, be manufactured by using electro-spinning. The pore sizes of the resorbable pouch may be 1-1000 µm, preferably 100-500 µm and even more preferably 200-250 µm. The pouch may be made of a polyester material, preferably PET. The pores of the pouch may have a diameter of less than about 1 mm.

The invention is also directed at a method for preparing a porous, bioresorbable, GDF-releasing calcium phosphate cement comprising:
providing a combination of a bioresorbable calcium phosphate material and a GDF as a biologically active agent and mixing said combination so that pores are formed upon hardening to produce a porous, bioresorbable, GDF-releasing calcium phosphate cement. Said pores may be elongated and have a diameter of about 100 µm to about 1 mm, preferably 100 µm to about 200 µm. The pores may have a length of more than 1 mm. Alternatively or additionally, the pores may be spherical pores with a diameter of less than 1 µm. The method may further comprise providing a cavity at the bone location, inserting a porous pouch into the cavity and administering the composition into said porous pouch, e.g., via a single passage to said cavity.

### Detailed Description of Various and Preferred Embodiments of the Invention

The present invention is directed at compositions adapted for use in the repair, regeneration, and augmentation of bone tissue. The composition preferably comprises a growth and differentiation factor, such as GDF-5 and GDF-3, and a biocompatible and bioresorbable calcium phosphate material. Preferably, the composition is cell-free. Upon hardening, the composition provides a resorbable material for new bone growth. A gas-forming agent may be included in the composition to promote formation of a desired pore structure or control porosity. The growth and differentiation factor(s) stimulate osteoinductive%steogenic activity of present or infiltrating progenitor or other cells. The compositions are in particular useful for osseous augmentation and regeneration of bone tissue, for example, in osteopenic or osteoporotic bone, as well as for tissue repair and reinforcement in bone fractures, dental implants, bone implants and prostheses, and the like.

Growth and differentiation factor-5 (GDF-5; also known as MP52, CDMP-1 or BMP-14) and growth and differentiation factor-3 (GDF-3; also known as Vgr-2) are members of the heterogeneous transforming growth factor β (TGF-β)/bone morphogenetic protein (BMP) superfamily. Members of this family are known for their role in the developmental events that dictate skeletal patterning. GDF-5 defines a distinct structural and functional subgroup of BMPs and shares 40-50% protein-sequence homology (38% sequence identity on the RNA level) with BMP-2 and BMP-7 (osteogenic protein-1, or OP-1). GDF-5 has a high binding affinity for the BMP receptor ALK-6 and is expressed in the prechondrogenic mesenchyme and can induce/promote the formation of the initial condensations in micromass cultures, while e.g., the widely used BMP-2, BMP-3 and activins act at the later steps of chondrogenesis (P. H. Francis-West, Development 126, 1305-1315 (1999)). Recombinant human (rh) GDF-5 could also be shown to stimulate mesenchyme aggregation and chondrogenesis. However, in contrast to BMP-2, osteoblastic MC3T3-E1 cells, which serve as osteogenic markers, did not respond to rh GDF-5, suggesting that GDF-5 may be relatively specific for chondrogenesis. Also, the receptor-binding profiles of GDF-5 are more limited than those of BMP-2 and BMP-7 and these receptors display a more restrictive tissue distribution (Spiro, Anat. Record 263,388-395 (2001)). GDF-5 and BMP-2 also show completely different temporal and spatial expression patterns in developing limbs, with GDF-5 having been attributed a role in the early stage of chondrogenesis and chondrocyte development. In cell culture studies, recombinant human GDF demonstrated to be considerably less potent in osteogenesis relative to other BMPs, in particular BMP-2 (Hoetten, Growth Factors 13, 65-74 (1996), (Depprich et al., Mund Kiefer Gesichtschir., 9(6): 363-8 (2005) as well as BMP-7 (OP-1) (Erlacher, J Bone Mineral Res. 13(3), 383-392 (1998)).

GDF-3's sequence identity on the RNA level to BMP-2 is, with 20% even lower than that of GDF-5. While other members of the BMP family are expressed at different embryonic stages and in different regions of developing bone, GDF-3 is expressed in the osteogenic zone of newly calcifying bone (Jones Mol. Endo 6(11), 1961-68 (1992)).

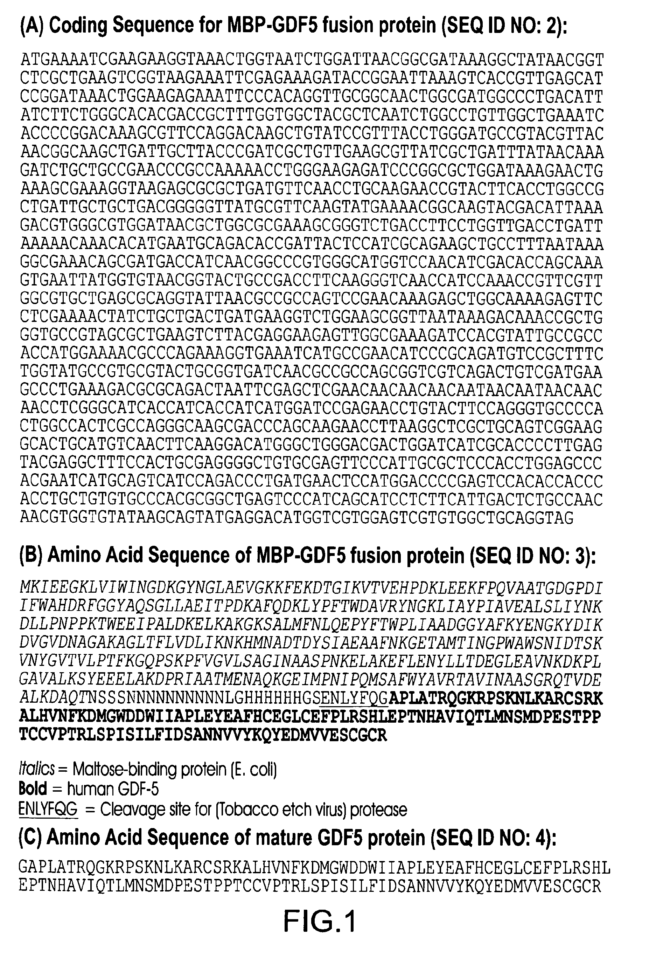

W093/1609 (see also U.S. Patent 6,120,760) describes a coding sequence of GDF-5. Generally, GDF-5 is expressed as a precursor which is composed of an amino terminal signal sequence and a carboxyterminal sequence of about 100 amino acids, which after cleavage presents the mature protein. The *Gdf5* gene from human can be found under accession no. X80915 (PRI 04-MAY-2005), the amino acid sequence can be found under Swiss-Prot: P43026 (modified 2007-08-21). Fig. 1(A) shows a coding region for GDF-5 and Fig. 1(B) the amino acid sequence of a precursor fusion protein of GDF-5, while Fig. 1(C) shows the amino acid sequence of a mature GDF-5.

Any "biologically active agent" according to the present invention furthers (ergo is an osteogenic biologically active agent) or induces (ergo is an osteoinductive biologically active agent) bone growth when provided as part of the composition of the present invention. Of particular interest are osteogenic and/or osteoinductive materials such as bone morphogenetic proteins such as BMP-2, BMP-4, BMP-5, BMP-6, BMP-7, BMP-10, in particular growth and differentiation factors (GDFs) such as GDF-6 and GDF-7, but in particular, e.g., recombinant human GDF-5 or GDF-3, transforming growth factors (e.g., TGF-β), and various other organic species known to further and/or induce bone formation. The biologically active agent may also comprise, in certain embodiments, bone-forming cells or bone-forming precursor cells.

A "growth and differentiation factor" (GDF) according to the present invention is any GDF, in particular GDF-5 and /or GDF-3, or a functional fragment or functional variant thereof that furthers and/or induces osteogenesis (are osteogenic and/or osteoinductive) when provided in the composition and method of the present invention. A "functional fragment" can, for example, comprise terminal and/or internal omissions compared to a wild type or a recombinant GDF produced from a cDNA of a wild type, as long as it furthers or induces osteogenesis when provided as part of the composition, kit and/or method of the present invention. A functional variant can, for example, contain one or more point mutations compared to the wild type. A functional variant may also be a functional fragment, that is, comprise one or more point mutations as well as terminal and/or internal omissions as long as the functional variant furthers and/or induces osteogenesis when provided as part of the composition and/or method of the present invention. Functional fragments and functional variants having this function, but whose functionality is reduced to about 70%, about 80%, or about 90% of a wild type, or enhanced by about 10%, about 20%, or about 30% compared to a wild type or recombinant GDF produced from a cDNA of a wild type, are also within the scope of the present invention. A functional fragment preferably comprises SEQ ID No: 1: MNSMDPEST. The GDF-5 and/or GDF-3 or functional fragment may be at least 50%, 60%, 70%, 75%, 80%, 85%, 90%, 95%, 96%, 97%, 98%, or 99% identical to the respective wild type sequence, that is, have these sequence identities (in %) with this wild type sequence.

The term "sequence identity" refers to a measure of the identity of nucleotide sequences or amino acid sequences. In general, the sequences are aligned so that the highest order match is obtained. "Identity" per se has recognized meaning in the art and can be calculated using published techniques. (See, e.g.: Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). While there exist a number of methods to measure identity between two polynucleotide or polypeptide sequences, the term "identity" is well-known to skilled artisans (Carillo, H. & Lipton, D., SIAM J Applied Math 48:1073 (1988)).

Whether any particular nucleic acid molecule is at least 50%, 60%, 70%, 75%, 80%, 85%, 90%, 95%, 96%, 97%, 98%, or 99% identical to a nucleic acid sequence encoding a specified GDF or a functional fragment thereof, can be determined conventionally using known computer programs such as DNAsis software (Hitachi Software, San Bruno, Calif.) for initial sequence alignment followed by ESEE version 3.0 DNA/protein sequence software (cabot@trog.mbb.sfu.ca) for multiple sequence alignments. An alternative sequence analysis program is provided under the name VECTOR NTI by Invitrogen.

Whether the amino acid sequence is at least 50%, 60%, 70%, 75%, 80%, 85%, 90%, 95%, 96%, 97%, 98%, or 99% identical to, for instance a wild-type ICD amino acid sequences, or a subportion thereof, can be determined conventionally using known computer programs such the BESTFIT program (Wisconsin Sequence Analysis Package, Version 8 for Unix, Genetics Computer Group, University Research Park, 575 Science Drive, Madison, Wis. 53711). BESTFIT uses the local homology algorithm of Smith and Waterman, Advances in Applied Mathematics 2:482-489 (1981), to find the best segment of homology between two sequences.

When using DNAsis, ESEE, BESTFIT or any other sequence alignment program to determine whether a particular sequence is, for instance, 95% identical to a reference sequence according to the present invention, the parameters are set such that the percentage of identity is calculated over the full-length of the reference nucleic acid sequence and that gaps in homology of up to 5% of the total number of nucleotides in the reference sequence are allowed.

The GDF according to the present invention, such as GDF-5 may be recombinantly produced, or, alternatively, purified from a protein composition. Preferably, the GDF of the present invention is produced recombinantely, e.g., in mammalian cell cultures. Bacterially-produced GDF is, however, in a certain embodiment of the invention, even more preferred. The GDF may be non-glycosylated and may have relatively low solublity. In certain embodiments the GDF remains on the carrier for, e.g., one year up to a lifetime, preferably one to ten years. As the person skilled in the art will appreciate, in certain applications, a shorter retention time may be preferable, e.g., about 1 year or less, while in other applications, a longer retention time may be preferable. Longer retention times allow in certain embodiments for low dosage administration regimes, such as from about 100 µg or less, up to 1 mg. The administration may be a single administration or may be repeated two or three times. Low dosage regimes reduce the negative side effects that have been associated with the use of BMPs such as BMP-2 and BMP-7, e.g., the induction of tumors of haemapoietic cells, ectopic ossification, and systemic antibody formation (Harwood et al., Expert Opin Drug Saf 4(1): 75-89 (2005)). The GDF may also be only partially glycosylated, that is less glycosylated than the respective wild type GDF. The GDF may be homodimeric, or may form a heterodimer with another BMP or with other members of the TGF-β superfamily, such as activins, inhibins and TGF-β-1 (e.g., a heterodimer composed of one monomer each of a BMP and a related member of the TGFβ superfamily). Examples of such heterodimeric proteins are described for example in Published PCT Patent Application WO 93/09229 (see also, e.g., U.S. Patent 6,593,109).

The compositions of the present invention may further comprise additional agents such as the Hedgehog, Frazzled, Chordin, Noggin, Cerberus, and Follistatin proteins. These families of proteins are generally described in Sasai et al., Cell 79:779-790 (1994) (Chordin); PCT Patent Publication W094/05800 (Noggin); and Fukui et al., Devel. Biol. 159:131-139 (1993) (Follistatin). Hedgehog proteins are described in W096/16668; W096/17924; and W095/18856. The biologically active agent may also include other soluble receptors, such as the truncated soluble receptors disclosed in PCT patent publication W095/07982. From the teaching of W095/07982, one skilled in the art will recognize that truncated soluble receptors can be prepared for numerous other receptor proteins, which are also encompassed within the present invention.

The amount of GDF useful herein is an amount effective to stimulate increased osteogenic and/or osteoinductive activity of infiltrating progenitor or other cells (a "bone formation effective amount"). In clinical applications such as for the treatment or prevention of fractures of a patient in need thereof, the effective amount will depend upon the size and nature of the defect being treated or the anticipated chance of fracture. Generally, the amount of protein to be delivered is in the range of from 1 µg to 100 mg, preferably less than 10 mg, less than 1 mg, less than 750 µg, or less than 500 µg. Most preferably, the CaP-GDF, is administered at relatively low doses of GDF, e.g., 500 µg or less of GDF, such as 400 µg, 300 µg, 200 µg, 100 µg, in particular less than 100 µg, including less than 75 µg, 50 µg, or 25 µg.

As described in the Examples, the GDF comprising calciumphosphate (CaP-GDF) composition may be injected into a fractured vertebral body, e.g., of a person having osteoporosis. The treatment regime may comprise in a preferred embodiment a single administration step or may comprise a series of administrations. Preventatively, the CaP-GDF composition can be injected into vertebral bodies adjacent to the fractured vertebral body to prevent post-implant fractures at such adjacent vertebral bodies. Such an injection can either be performed after the treatment of the fractured vertebral body has progressed to a point at which new bone has formed, e.g., the total bone volume has increased about 10%, about 20%, about 30%, about 40%, or about 50% at a local area or alternatively, e.g., two weeks or more, e.g., four week to six weeks, two or three months, or longer after the initial treatment regime of the fractured vertebral body has been completed. However, as the person skilled in the art will recognize, the timing of such a subsequent injection will depend on factors such as the overall health of the patient, the condition of the bone material of the patient, with heavily damaged bone material advocating for a sooner second administration. Alternatively, the preventative injection of the CaP-GDF composition is performed at the time the fractured vertebral body is treated. CaP-GDF may also be administered purely preventatively, e.g., solely based on a high likelihood of fracture. The likelihood of fracture can, e.g., be determined, e.g., by the standard deviation of the bone mineral density (BMD) measured in a weakened bone similar to that that will be determinative for bisphosphonate administration. The value at which treatment is warranted depends on a multitude of factors such as age, gender, previous fractures, nicotin consumption, etc. For example, treatment may be warranted in an otherwise healthy woman over 75 or a man over 85 at a standard deviation as low as -2.0, but in man between 60 and 70 only at a standard deviation of about -4.5. However, if the man in the latter example smokes, the value may be lowered to -3.5. In a patient with rheumatoid arthritis, which is subject to a prolonged steroid treatment, a preventative treatment might be warranted at a standard deviation of -1.5. In particular during a preventative treatment regime, the CaP-GDF may be administered in combination with other bone strengtheners, e.g., bisphosphonates such as alendronate or the parathyroid hormone fragment teriparatid.

In a preferred embodiment, kits are provided comprising the elements of the present invention. For example, such a kit may comprise, in one container, the calcium phosphate material, optionally in dried form, optionally admixed with the biologically active agent which may also be provided in dried, e.g., freeze-dried, form. Another container may include instructions how to admix and/ or use the materials in the first container. The biologically active agent may alternatively be provided in a further separate container. Here the biologically active agent may be provided in dried form or may be in solution that, e.g., may also comprise a buffer material. In certain embodiments the kit may also comprise administration tools for, e.g., kyphoplasty. Those tools may, e.g., include injection needles specifically adjusted to the components of the composition that shall be administered to a patient.

The CaP-GDF composition, kits, and methods of the present invention are, in a preferred embodiment, used in conjunction with vertebroplasty and/or kyphoplasty. In a classical form of these two approaches to bone regeneration, PMMA cement is injected into the fractured vertebral body to render it mechanically more resistant. In kyphoplasty pressurized balloons are used to increase the height of the affected vertebral body. Published US Patent application 20060195115 describes for example, an apparatus for kyphoplasty. US Patent 6,423,083 describes a removable balloon for use in compressing cancellous bone and marrow against the inner cortex of bones whether the bones are fractured or not. Published US Patent application 20060182780 describes a method of manufacturing a resorbable balloon designed to contain bone cement for vertebroplasty or kyphoplasty applications. As the person skilled in the art will appreciate, the compositions, kits, and methods can also be employed in the context of spondylodesis.

The conversion reaction that produces eventually the hardened material may be initiated by adding distilled water to a mixture of the dry precursor components to form a thick hydrated precursor in the form of a paste or putty. "Injectable" hydrated precursor material has a consistency appropriate for delivery through, e.g., an 18 or 9 gauge injection needle. In particular, the injectable material may have a consistency that allows delivery through an injection needle having an inner diameter of 1 to 10 mm, preferably 2 to 3 mm, such as 2.2 mm. The hydrated precursor material undergoes conversion to a hardened calcium phosphate, optionally with the assistance of a promoter material such as dicalcium phosphate dihydrate (DCPC). Preferably the hardening is completed in less than 12 hours, preferably 5 hours, more preferably about 5 minutes to about 60 minutes, and even more preferably about 10 minutes to about 30 minutes. The time course of conversion may be adjusted to the surgical procedure employed, but should, in any event, be fast enough to have surgical utility. The hardening does, in a preferred embodiment, proceed *in situ.* However, alternatively, the material may, e.g., be pre-hardened outside the body, loaded with the desired biologically active agent, and implanted at a later time. This approach is useful in those situations where custom shapes are not essential, and where production of large numbers of implants is desired.

As used herein, a "calcium phosphate material" means a synthetic bone substitute material comprising calcium phosphate as the primary component. Suitable calcium phosphate-based materials are well known in the art and include, without limitation, amorphous calcium phosphate, hydroxyapatite, tricalcium phosphate, such as a/ß-tricalcium phosphate, and fluorapatite. In a preferred embodiment, the calcium phosphate material is an apatitic calcium phosphate solid having a calcium-to-phosphate (Ca/P) ratio comparable to naturally occurring bone minerals. However, in certain embodiments, the calcium phosphate material will be "calcium deficient", with a calcium to phosphate ratio of less than or equal to 1.5 as compared to the ideal stoichiometric value of approximately 1.67 for hydroxyapatite. Such materials may be produced using a combination of amorphous calcium phosphate as a reactant, a promoter, and an aqueous liquid to form a hardening paste. In an alternative embodiment, the calcium phosphate material is produced by solid-state acid-base reaction of crystalline calcium phosphate reactants to form crystalline calcium phosphate solids. In a further embodiment, monocalciumphosphate hydrate is mixed with tricalcium phosphate, such as β-tricalcium phosphate and water, to produce brushite and water.

A "gas-forming agent" refers to a gaseous substance or a substance, which produces bubbling, foaming, or liberation of a gas. These substances include, but are not limited to, hydrogen, carbon dioxide, air, nitrogen, helium ,oxygen, or argon.

As used herein, "amorphous" (also known as "X-ray amorphous") means a material with significant amorphous character. Significant amorphous character contemplates greater than 75% amorphous content, preferably greater than 90% amorphous content, and is characterized by a broad, featureless X-ray diffraction pattern.

The term "biocompatible," as used herein, means that the material does not elicit a substantial detrimental response in the host. There is always concern, when a foreign object is introduced into a living body, that the object will induce an immune reaction, such as an inflammatory response that will have negative effects on the host. For example, although hydroxyapatite is generally considered to be "biocompatible", significant inflammation and tissue necrosis have been observed when crystalline hydroxyapatite microcarriers are inserted intramuscularly in animals (see, for example, Jintema et al., Int. J. Pharm. 112:215 (1994)).

"Bioresorbable" refers to the ability of a material to be resorbed *in vivo*. The resorption process involves elimination of the original implant materials through the action of body fluids, enzymes, or cells. Resorbed calcium phosphate may, for example, be redeposited as bone mineral, be otherwise reutilized within the body, or excreted. "Strongly bioresorbable," as that term is used herein, means that at least 80% of the total mass of material implanted intramuscularly, subcutaneously, or into bone is resorbed within one year. In preferred embodiments, the material will be resorbed within nine months, six months, three months, and in certain embodiments, ideally one month.

A composition of the present invention is said to be "cell-free" if, prior to administration, it does not contain any bone-forming cells, such as progenitor cells, stem cells, and/or osteoblasts.

An "effective amount" of a gas-forming agent is an amount sufficient to effect the formation of a desired porosity and a desired pore structure upon hardening, and will depend upon the calcium phosphate material being used. Generally, the amount of gas-forming agent is added in a range of from about 1 to 90 percent by weight, preferably about 1 to 50 percent by weight, and more preferably about 10 to 40 percent by weight.

A "porosity" that is desirable to further resorption generally entails pores having a diameter of more than about 100 µm, preferably about 100 to about 200 µm. However, larger pores such pores having a diameter of, e.g, 500 µm are also within the scope of the present invention. However, in certain embodiments the hardened material has, additionally or alternatively, small pores, such as pores of 1 µm or less, which, while allowing a certain degree of accessiblity, confer a high degree of mechanical strength. In terms of "pore structure" interconnecting pores that further resorption by facilitating cell migration throughout the material and provide access for unobstructed vascularisation are preferred.

As used herein, "macroporous" refers to a hardened calcium phosphate material having pores of sufficient diameter to permit cell migration and infiltration. In a preferred embodiment, the macroporous material formed in accordance with the present invention has a pore diameter of greater than 10 µm or greater than 30 µm, more preferably between about 30 and 200 µm, and most preferably between about 50 and 100 µm in diameter. The macroporous material of the present invention preferably facilitates cell migration and infiltration for secretion of extracellular bone matrix, as well as enhancing cell-matrix interactions. In a preferred embodiment, pores of the hardened calcium phosphate material are interconnected to further enhance cell migration and infiltration throughout.

As used herein, "microporous" refers to a hardened calcium phosphate material having pores of a diameter of less than about 100 µm, generally between about 1 and about 100 µm. "Nanoporous" refers to a hardened calcium phosphate material having pores of a diameter of less than 1 µm.

An "effective amount" of a growth and differentiation factor or a composition comprising such a growth and differentiation factor is an amount sufficient to stimulate increased osteogenic activity of present or infiltrating progenitor or other cells ("bone formation effective amount"). The amount will depend upon the size and nature of the defect being treated, as well as the composition of the calcium phosphate material being employed. Generally, the amount of biologically active agent to be delivered is in a range from about from 1 µg to 100 mg, preferably less than 10 mg, less than 1 mg, less than 750 µg or less than 500 µg. Most preferably, the CaP-GDF, is administered at relatively low doses of GDF, e.g., 500 µg or less of GDF, such as 400 µg, 300 µg, 200 µg, 100 µg, in particular less than 100 µg, including less than 75 µg, 50 µg, or 25 µg.

An "effective amount" of an additional material such as a polymeric structure is an amount sufficient to impart the desired mechanical or chemical property to the composite.

"Hardening" refers to the process by which the malleable calcium phosphate composition is transformed into a hardened calcium phosphate material. The calcium phosphate material/the composition comprising the calcium phosphate material is considered to be "hardened" when it is a substantially non-formable solid. Such a hardened calcium phosphate material has minimal compressibility and tends to undergo plastic as opposed to elastic deformation. A hardened composition according to the present invention is also referred to herein as a "cement" or "matrix."

The "elastic modulus" is a parameter that represents the stiffness of a material. The elastic modulus is used herein to represent a force per unit surface area and is therefore given in Pascal (1 bar = 10⁵ Pa), or Megapascal (1 MPa). Cortical bone has a higher elastic modulus (e.g., about 18,000 MPa) than cancellous bone (e.g., about 150 MPa). The elastic modulus is heavily influenced by age, presence of osteoporosis, skeletal site, etc. Treatment with the composition of the present invention may result in an about 2 fold, 5 fold, 6 fold, 7 fold, 8 fold, 9 fold, 10 fold, or higher increase in the elastic modulus. A desirable elastic modulus in bone treated with the composition described herein will depend on the skeletal site treated. However, in certain embodiments, depending on the bone material treated , the desirable elastic modules of the treated bone is about 50 to about 1500 MPa, preferably about 100 to about 150 MPa or about 500 to about 1200 MPa (see also, Baroud and Boher, Joint Bone Spine 73:144-150 (2006).
"Compression strength" refers generally to the amount of pressure that can be applied to a material without causing damage. Cortical bone has been reported to have a compression strength of about 100 MPa, while osteoporotic cancellous bone has been reported to have a compression strength of about 4 MPa Baroud and Boher, Joint Bone Spine 73:144-150 (2006).

### The Calcium Phosphate Material

Calcium phosphate component of the present invention may be any biocompatible, calcium phosphate material known in the art (see, e.g, U.S. Patent 6,027,742 and U.S. Pat. No. 5,650,176). Hardening of the calcium phosphate material may be achieved by any one of a variety of methods and using any suitable starting components. For example, the hardening of calcium phosphate material may be initiated using a combination of a calcium phosphate material as a reactant, a promoter and an aqueous liquid to form a hardening paste. Alternatively, the calcium phosphate cement may be produced by solid-state acid-base reaction of crystalline calcium phosphate reactants to form crystalline calcium phosphate solids. Other methods of making calcium phosphate matrix are known in the art.

The calcium phosphate material may, for example, be crystalline hydroxyapatite (HA). Crystalline HA is described, for example, in U.S. Pat. Nos. Re. 33,221 and Re. 33,161 to Brown and Chow, both of which are herein incorporated by reference. The Brown and Chow Patents teach preparation of calcium phosphate remineralization compositions and of a finely crystalline, non-ceramic, gradually resorbable hydroxyapatite carrier material based on the same calcium phosphate composition. A similar calcium phosphate system, which consists of tetracalcium phosphate (TTCP) and monocalcium phosphate (MCP) or its monohydrate form (MCPM), is described by Constantz et al. in U.S. Pat. Nos. 5,053,212 and 5,129,905. In this embodiment, the calcium phosphate material is produced by solid-state acid-base reaction of crystalline calcium phosphate reactants to form crystalline hydroxyapatite solids.

Crystalline HA materials may be prepared such that they are flowable, moldable, and capable of hardening *in situ* (see U.S. Pat. No. 5,962,028 to Constantz). These HA materials (commonly referred to as carbonated hydroxyapatite) can be processed by combining the wet and dry reactants to provide a substantially uniform mixture, shaping the mixture as appropriate, and allowing the mixture to harden. Alternatively, precursor reaction mixtures can be administered to the surgical site and hardened and/or shaped *in situ.*

During hardening, the mixture crystallizes into a solid and essentially monolithic apatitic structure.

The reactants will generally comprise a phosphoric acid source substantially free of unbound water, an alkali earth metal, particularly calcium, source, optionally crystalline nuclei, particularly hydroxyapatite or calcium phosphate crystals, calcium carbonate, and a physiologically acceptable lubricant, such as water, which may have various solutes. The dry ingredients may be pre-prepared as a mixture and subsequently combined with the liquid ingredients under conditions in which substantially uniform mixing occurs.

The phosphoric acid source may be any partially neutralized phosphoric acid, particularly up to and including complete neutralization of the first proton as in calcium phosphate monobasic. Alternatively or additionally, it can comprise orthophosphoric acid, possibly in a crystalline form, that is substantially free of uncombined water. Calcium sources will generally include counterions such as carbonate, phosphate or the like, particularly dual sources of calcium phosphate and phosphate such as tetracalcium phosphate or tricalcium phosphate, such as a/β-tricalcium phosphate.

The various dry components may be combined prior to the addition of the wet components. Mixing combines the ingredients and can be used to regulate the extent of the inter-ingredient reactions. Any or all of the dry ingredients may be added prior to the initiation of mixing or prior to the completion of mechanical mixing. After mixing, the mixture is allowed to anneal while remaining quiescent, followed by an extended period of time during which the mixture hardens.

The resorption rate of the hardened CaP composition can be adjusted by providing calcium phosphate phases that resorb at different speeds. For example, larger, less quickly resorbing particles, such as HA particles, can be embedded in fast resorbing phases such as brushite or amorphous calcium phosphate or vice versa. Preferably the size of certain particles and percentile of each phase should be chosen so that slower resorbing particles have a mean distance from each other of more than 50 µm, preferably more than 100 µm, and even more preferably more than 200 µm. The larger, slower resorbing particles may, in certain embodiments, additionally comprise a pore structure in the submicrometer range, which can serve as a "depository" for biologically active agents such as GDF-5 and/or GDF-3. Such pores may comprise fast resorbing calcium phosphate material and thus mediate, on the one hand, a fast release of the biologically active agent(s), but on the other hand protect the biologically active agent(s) from the chemical reactions that take place during the hardening of the composition. The depository will allow an extended release of biologically active agent in the cement formed, while autologous bone formation rapidly proceeds throughout the surrounding fast resorbing material. Further avenues of influencing the resorption rate are pore structure, porosity, and size of pores of the hardened composition. These parameters can be used to influence the total amount of resorbing material as well the surface/volume ratio, which also affects the resorption kinetics.

### Gas-Forming Agent

In certain embodiments, the compositions include a gas-forming agent, which will facilitate the process for producing a calcium phosphate matrix or scaffold material. The gas-forming agent may in particular be used to create pores in the hardened composition, in particular interconnecting pores for vascularization and cell ingrowth. In other embodiments, it will be helpful in producing a composition whose calcium phosphate material "self-granulates" and disperses into hardened macrogranules or macroparticles under physiological conditions (i.e., post-administration). The addition of a gas-forming agent to the calcium phosphate materials may alter the biological, chemical, and mechanical properties of the material and may enhance its therapeutic utility in certain applications. The gas-forming agent of the present invention may be any pharmaceutically acceptable substance which produces bubbling or liberation of a gas at physiological temperatures and/or pressures.

Methods for producing calcium phosphate materials for use with seeded compositions often suffer from limited injectability due to granule formation during production or preparation for administration in the syringe.

An added gas-forming agent may mitigate these drawbacks of the prior art. The gas-forming agent may be added to the other components of the composition (e.g., calcium phosphate material and any supplementary materials) to cause gas-foaming or -bubbling under specific conditions (i.e., physiological temperatures and/or pressures). The gas formation may assist in producing a desirable porosity and pore structure in the calcium phosphate material as discussed above upon injection or implantation *in vivo*.

In one embodiment, the gas-forming agent is a gas which is dissolved in the hydrated calcium phosphate material. The gas may be dissolved in the material under pressure, i.e., by subjecting the composite material to a pressurized atmosphere of the gas, but which is inert to the cementing reaction. The gas is then liberated upon exposure to physiological temperatures (i.e., upon injection or implantation), due to the decrease in gas solubility with increased temperature. Under these circumstances, the gas dissolution occurs only during hardening in *vivo*, and not prior to administration. By way of example only, suitable gases include hydrogen, carbon dioxide, air, nitrogen, helium, oxygen, and argon.

In another embodiment, the gas-forming agent is a volatile liquid which vaporizes at physiological temperatures. The gas-forming agent may also be a liquid that decomposes forming gas, such as H₂O₂.

In yet another embodiment, the gas-forming agent is a solid material which liberates gas upon dissolution. For example, sodium bicarbonate evolves carbon dioxide gas as it converts to an unstable carbonic acid intermediate, which subsequently evolves carbon dioxide and water.

### Bone-Forming Cells

The composition of the present invention is in many embodiments preferably cell-free and will only be populated with cells, in particular the patient's own bone-forming cells *in situ*. However, in certain embodiments, in order to optimize ossification, the calcium phosphate composition may be seeded with bone-forming cells, such as progenitor cells, stem cells, and/or osteoblasts. This is most easily accomplished by placing the calcium phosphate composition in contact with a source of the patient's own bone-forming cells. Such cells may be found in bone-associated tissue, blood or fluids, including exogenous fluids which have been in contact with bone or bone materials or regions, including the periosteum, cancellous bone or marrow. When used in conjunction with devices such as screws and pins, the introduction of which into bone is accompanied by breach of the periosteum and/or bleeding, no further seeding is required. For plates, which oppose only cortical bone, induction of a periosteal lesion which will contact the device is generally recommended. In yet other embodiments, it will be useful to surgically prepare a seating within the bone by removing a portion of cortical bone at the implant site. Bone-forming cells harvested from the patient may be introduced into the graft to augment ossification. Use of non-autologous bone cells is also within the scope of the invention if the desired amount of bone regeneration occurs prior to host rejection of the bone-forming cells. Thus, cells or tissues obtained from primary sources, cell lines or cell banks may all be useful in certain embodiments. See, U.S. Pat. No. 6,132,463 to Lee et al., which is incorporated herein by reference.

### Supplementary Material

The composite material of the present invention may be prepared by combining the calcium phosphate material, biologically active agent and, optionally, a gas-forming agent, with a selected supplementary material. The calcium phosphate material may serve as the reinforcing material, the matrix or both. The calcium phosphate material in its initial hydrated form typically maintains a pH of about 6-7 and is therefore compatible with a wide range of additives without deleterious effect. The supplementary material is selected based upon its compatibility with calcium phosphate and the other components and its ability to impart properties (biological, chemical, or mechanical) to the composite, which are desirable for a particular therapeutic purpose. For example, the supplementary material may be selected to improve tensile strength and hardness, increase fracture toughness, provide imaging capability, and/or alter flow properties, and setting times of the calcium phosphate material. For example, the supplemental material may be added to provide for an elastic modulus of 100 to 1000 MPa in the hardened composition. In another example, the supplementary material may be designed to contain the calcium phosphate material at the desired site.

In certain embodiments, the supplementary material may be added to the calcium phosphate composition in varying amounts and in a variety of physical forms, dependent upon the anticipated therapeutic use. For example, the supplementary material may be in the form of solid structures, such as sponges, meshes, films, fibers, gels, filaments, or particles, including macro- and nanoparticles. The supplementary material itself may be a composite. In a preferred embodiment, the supplemental material is a polymeric structure, in particular in the form of a microscaffold. Such a microscaffold may be between 500 µm and 1 mm in size, and contain pores that prefably have a diameter of about 10 to about 1000 µm, preferably about 30 to about 500 µm, preferably about 100 to about 200 µm. In certain preferred embodiments it is coated with a biocompatible material, preferably a nanocrystalline calcium phosphate. The microscaffold may be made of a variety of materials. In a preferred embodiment the microscaffolds are made from resilient material such as chitosan and/or a poly-lactic-acid/ poly-e-caprolactone (PLA-PCL) copolymer. The resilience of these materials allows the microscaffold to have a size larger than the inner diameter of a typical injection needle, which generally is from 2 mm to 3 mm but may be up to 10 mm. The supplementary material also includes, in certain embodiments, hollow beads, which may provide more "porelike" structures. In other embodiments, the supplemental material may also come in the form of non-hollow, nano- or macroparticles.

The supplemental material may also be a porous pouch into which the composition comprising the calcium phosphate material is filled. The pouch may be inserted into a cavity that is created in the bone location through, e.g., a small tube of, e.g., 3 mm to about 1 cm in diameter. The non-inflated porous pouch may be fed through the tube into the cavity and may, when in place, be filled with the composition via, e.g., a needle, catheter or similar. In certain embodiments providing a single cavity may suffice. The pouch may generally be from about 1 cm to about 4 cm in diameter, and is preferably of rounded shape, e.g., round, spherical or elliptical. Upon being filled with the composition it may, preferably, take the shape of the cavity in which it was deposited. The pouch preferably will allow fluids to enter and/or exit and/or will allow the in-growth and through-growth of blood vessels, fibrous tissue and bony trabeculae. However, the porosity of the pouch should be such that the calcium phosphate material is retained therein, while the GDF can exit at a desirable rate. Depending on the calcium phosphate material used, the pores have preferably a diameter of less than 5 mm, but preferably less than about 3 mm, less than about 2 mm, less than about 1 mm, less than about 0.5 mm or even less than about 0.25 mm, less than about 0.1 mm, less than about 0.05 mm, but generally not lower than 0.01mm. The size of the pores is preferably selected to allow tissue in-growth and GDF exit while containing the calcium phosphate material in the pouch. Generally, the pores will be chosen as small as possible so as to prevent leakage of the calcium phosphate material, since leaking calcium phosphate material could impinge upon nerves or blood vessels. However, at least one pore opening may be larger than the rest to allow the pouch to be filed. Preferably even this pore opening is less than 5 mm in diameter. However, its diameter will be depend on the needle, catheter or similar used to fill the pouch.

The size and density of the pores determine the ease or difficulty with which materials may pass through. For instance, very small pores (<0.5 mm) generally would prohibit passage of all but the smallest particles and liquids. A combination of pores sizes and/or densities may allow for a "controlled" release of the GDF, i.e., a release of GDF over time. For example, two or more pore sizes could be combined to allow, e.g., for an initial peak in release of GDF, followed by a low, but constant release over, e.g., a period of at least one month, at least two months or preferably at least three months. In certain embodiments at least a fraction of the GDF remains in the pouch for, e.g., one year up to a lifetime, preferably one to ten years. As the person skilled in the art will appreciate, in certain applications, a shorter retention time may be preferable, e.g., about 1 year or less, while in other applications, a longer retention time may be preferable. Longer retention times allow in certain embodiments for low dosage administration regimes, such as from about 100 µg or less, up to 1 mg. The pore size and density may be controlled in the manufacturing process.

The material of the pouch is preferably biocompatible, flexible and has good tensile strength. The material may be a woven, knitted or braided fabric made of resorbable and/or nonresorbable thread. The pouch is preferably formed of a plastic material, such as, but not limited to, a polyester material, e.g., Polyethylene Terephthalate (PET), but may be made of any material that would be compatible with the uses described above (see also U.S. Patent 7,226,481). The person skilled in the art will appreciate that the pouches may be combined with one or more other supplemental materials described herein.

The supplementary material may be a particulate or liquid additive or doping agent which is intimately mixed with the resorbable calcium phosphate. When intimately mixed with a calcium phosphate material, the supplementary material may interfere on a macroscopic level with the cementing reaction. This may occur with the supplementary material coating a percentage of the cement particles, allowing a weak cementing reaction to occur with the coated particles. Alternatively, the liquid or solid may cause physical separation between the reactive species resulting in focal areas of cement formation (or granules). The supplementary material may serve as a matrix for the calcium phosphate, which is embedded or dispersed within the matrix. Alternatively, the calcium phosphate may serve as a matrix for the supplementary material, which is dispersed therein. The supplementary material may be applied as a coating onto a calcium phosphate body, for example, as a post-fabrication coating to retard resorption time or otherwise affect the bioceramic material properties. Lastly, the supplementary material may be coated with the calcium phosphate composition.

The supplementary materials are preferably biocompatible, that is, there is no detrimental reaction induced by the material when introduced into the host. In many instances, it is desirable that the supplementary material also be bioresorbable. The supplementary material may have an affinity for calcium, phosphate or calcium phosphates which will enhance the strength of the calcium phosphate/ supplementary material interface. The affinity may be specific or mediated through non-specific ionic interactions. The supplementary material may be bioerodible, that is, susceptible to degradation over time, usually months (while a a "non-erodible" or "poorly erodible" material is, if at all, degraded over decades or at best, many years). By way of example only, suitable bioerodible polymers for use as a matrix in the composition include, but are not limited to, collagen, glycogen, chitin, celluloses, starch, keratins, silk, nucleic acids, demineralized bone matrix, derivativized hyaluronic acid, polyanhydrides, polyorthoesters, polyglycolic acid, polylactic acid, and copolymers thereof. In particular, polyesters of [alpha] hydroxycarboxylic acids, such as poly(L-lactide) (PLLA), poly (D, L-lactide) (PDLLA), polyglycolide (PGA), poly (lactide-co-glycolide (PLGA), poly (D, L-lactideco-trimethylene carbonate), and polyhydroxybutyrate (PHB), and polyanhydrides, such as poly (anhydride-co-imide) and co-polymers thereof are known to bioerode and are suitable for use in the present invention. In addition, bioactive glass compositions, such as compositions including SiO₂, Na₂O, CaO, P₂O₅, A₁₂O₃ and/or CaF₂, may be used in combination with the calcium phosphate composition of the invention. Other useful bioerodible polymers may include polysaccharides, peptides, and fatty acids.

Bioerodible polymers are advantageously used in the preparation of bioresorbable hardware, such as but not limited to intermedulary nails, pins, screws, plates, and anchors for implantation at a bone site. The bioresorbable fiber may be in the form of whiskers which interact with calcium phosphates according to the principles of composite design and fabrication known in the art. Such hardware may be formed by pressing a powder particulate mixture of the calcium phosphate and polymer. Alternatively, the calcium phosphate matrix may be reinforced with PLLA fibers, using PLLA fibers similar to those described by Tormala et al., which is incorporated herein by reference, for the fabrication of biodegradable self-reinforcing composites (Clip. Mater. 10:29-34 (1992)).

Bioresorbable polymers may also be used in the preparation of bone glues or putties for use in load-bearing situations. Supplementary materials may be added to the composite to increase compressibility and load-bearing properties of the bone glue. In particular, carbon fibers or other reinforcing fibers may be added to the composite. In the production of fiber-reinforced bone substitute glues, it may be advantageous to plasma-etch the fibers to improve the quality and strength of the calcium phosphate/fiber interface. Calcium phosphate may also be hardened at 37°C, pulverized or otherwise fragmented, and mixed with known binders such as bone glues, cements, fillers, plasters, epoxies, other calcium phosphates, or gels such as, but not limited to, calcium sulfate, alginate, collagen, or commercially available products such as Endobone (Merck), Hapset (Lifecore Biomedical), SRS(R) (Norian), Bonesource(R) (Leibinger), Collograft(R) (Zimmer), Osteograf(R) (CereMed), and Simplex(R) (Howmedica). For applications in which hardened calcium phosphate will be dispersed within the binder substance, most often the binder will be prepared by methods known to the art and mixed with the particulate calcium phosphate in approximately equal volumes, although actual proportions will be varied in ways known to the art to produce compositions of desired consistency, workability, and adherence.

In yet another embodiment, braided sutures, typically prepared from polyester, maybe coated with the calcium phosphate composition of the invention, to improve their biocompatibility. Coated sutures may be prepared by dipping the suture into a slurry containing the calcium phosphate material. The affinity of the suture for the calcium phosphate coating may be improved by surface treating either the suture, the calcium phosphate particle or both. Surface treatments include plasmaetching and/or chemical-grafting.

In other embodiments, a composite is provided comprising the calcium phosphate material and a non-resorbable or poorly resorbable material. Suitable non-erodible or poorly erodible materials include dextrans, polyethylene, polymethylmethacrylate (PMMA), carbon fibers, polyvinyl alcohol (PVA), poly (ethylene terephthalate) polyamide, bioglasses, and those compounds listed previously for use in bone glues or putties.

Another use is to permanently imbed useful objects, such as a pin or reinforcing mesh, into bone itself. The object serves as an anchor for the stable attachment to natural bone. This is particularly useful in the attachment of ligaments and tendons to bone. Objects comprising bioresorbable and ossifying calcium phosphate and a suitable non-resorbable hardware may be placed into a bone and further secured with additional calcium phosphate material or composite material in a bone glue formulation. The hardware then becomes embedded into the bone following reossification of the calcium phosphate material.

In yet another embodiment of the invention, a composition is prepared by blending the calcium phosphate material with an additive which alters the resorption properties, setting time and/or flow characteristics of the composition. For example, silicone oil or other lubricating polymers or liquids may be added to the composition to improve the flow characteristics of the composition for delivery to the host by syringe. The lubricant is preferably biocompatible and capable of rapid leaching from the bone-substitute material composite following solidification of the calcium phosphate composition *in vivo*. Suitable lubricants include, by way of example only, polymer waxes, lipids, and fatty acids. Lubricants may be used in a concentration of about 0.1 to about 30 wt %.

In yet another embodiment of the invention, a cement, ergo a hardened composition, is prepared by admixing to the composition a liquid, which will, together with the calcium phosphate material form a hardening solid phase, for example, partially neutralized phosphoric acid and calcium carbonate. During this reation CO₂ may form. Alternatively, monocalciumphosphate anhydrite, a/ß-tricalcium phosphate, and water may be combined and pores may be introduced via the mixing process.

Pores having a diameter of more than 100 µm can be achieved by the production of gas bubbles, e.g., by decomposing H₂O₂ into water and oxygen. Here H₂O₂ may be added to the composition at a concentration of 4% and at a ratio of 10:3. The size of the resulting bubbles, and thus the size and length of the pore canals which result from the escaping bubbles, can be adjusted by the concentration of H₂O₂ und the viscosity of the mixture.

Calcium phosphate comprising nanostructures can be produced, e.g., via original compositions comprising powders in the nanometer range. The agglomeration of these nanopowders and the resulting nanopores can be regulated by the production process of the powders (e.g., "pulse combustion", IBUtec, Weimar, Germany). Larger pores (but preferably smaller than 1 µm) can be provided by producing coarser powders from these agglomerates. Here, the particular size of the powder can also be adjusted via the production process. By means of a thermal treatment the composition of the phases can be adjusted, e.g., by a transformation of hydroxylapatite to a/β-tricalcium phosphate. A thermal treatment in which sintering temperatures are exceeded will result in spherical pores depending on the parameters of the thermal treatment.

The combination of prestructured, in particular nanoporous, powders, and gas-forming agents, may result in materials that combine nanoporosity and macroporosity.

The following examples detail presently preferred embodiments of the invention. Numerous modifications and variations in practice thereof are expected to occur to those skilled in the art upon consideration of these descriptions. Those modifications and variations are believed to be encompassed within the claims appended hereto. These examples do not in any way limit the invention.

### EXAMPLES

### Example 1:

Forty-eight (48) aged, osteoporotic sheep were subdivided into 8 groups and treated with different dosages of non-glycosylated recombinant human GDF-5 (Fig 1. (C)) (Hortschansky et al., Hans-Knöll Institute, Jena, Germany) via a fusion protein precursor (Fig. 1 (B)) from the nucleotide sequence shown in Fig. 1(A) and non-glycosylated recombinant human BMP-2 (Hortschansky et al., Hans-Knöll Institute, Jena). Groups 1, 3, 5, and 7 were treated with a minimal dosage of a composition comprising hydroxyapatite (HA) (CAMCERAM, HA protein coating powder, CAM Implants, B.V. Leiden, The Netherlands)) and rhBMP-2 and rhGDF-5, respectively, while groups 2, 4, 6, and 8 were treated with the maximal dosages of these compositions. A control (vertebral body not treated) and a HA only group in which the vertebral body was only treated with HA, were also included in the study. The experimental design is depicted in Table 1, which indicates that observations were made short-term (three months) and long-term (nine months). Five (5) mg HA particles were used per 100 µl osteogenic factor containing serum.

**Table 1**

| **Growth Factor** | **Dose [µg]** | **Time Period [months]** |
|---|---|---|
| rhBMP-2 | 2 (minimal) | 3 (short) |
| | | 9 (long) |
| | 20 (maximal) | 3 |
| | | 9 |
| rhGDF-5 | 10 (minimal) | 3 |
| | | 9 |
| | 100 (maximal) | 3 |
| | | 9 |

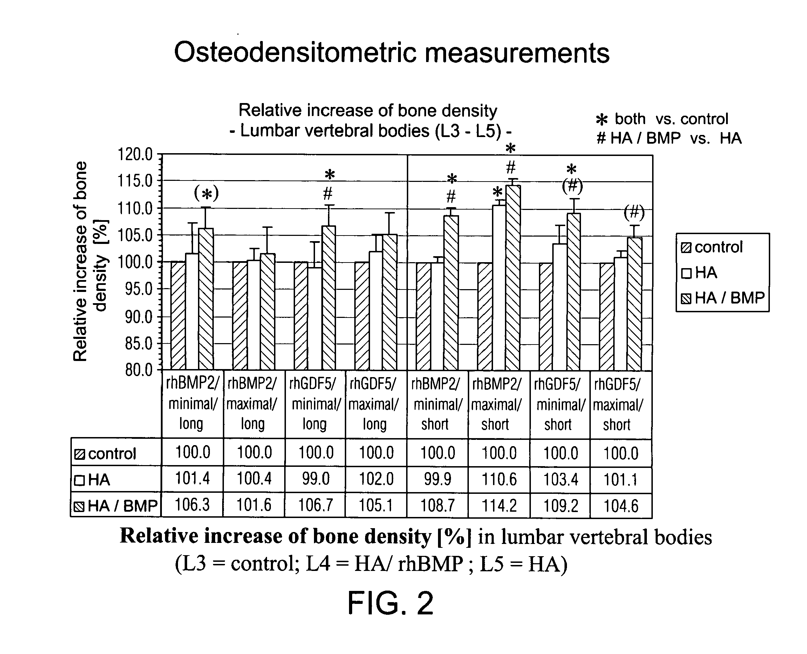

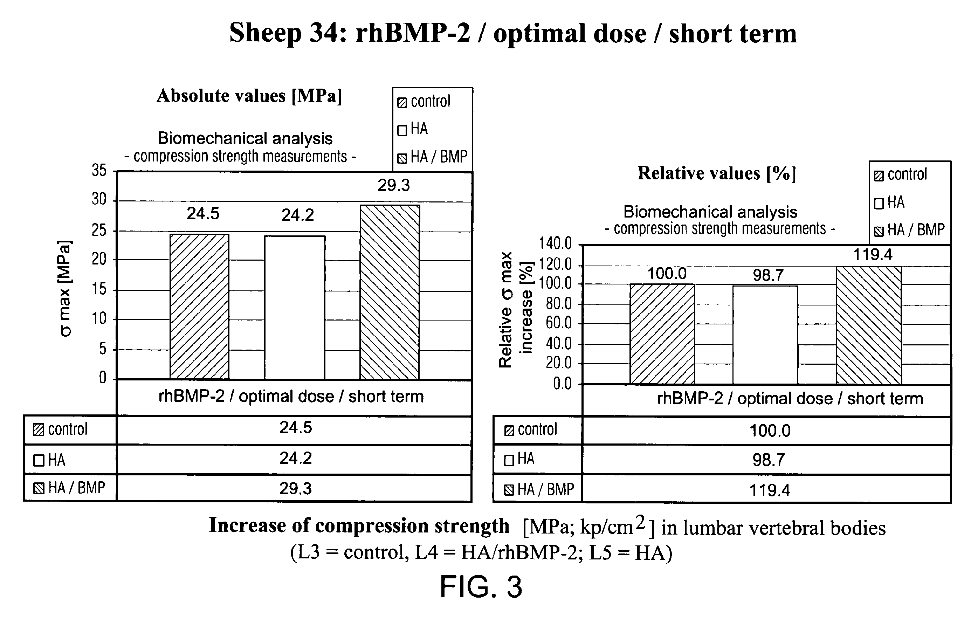

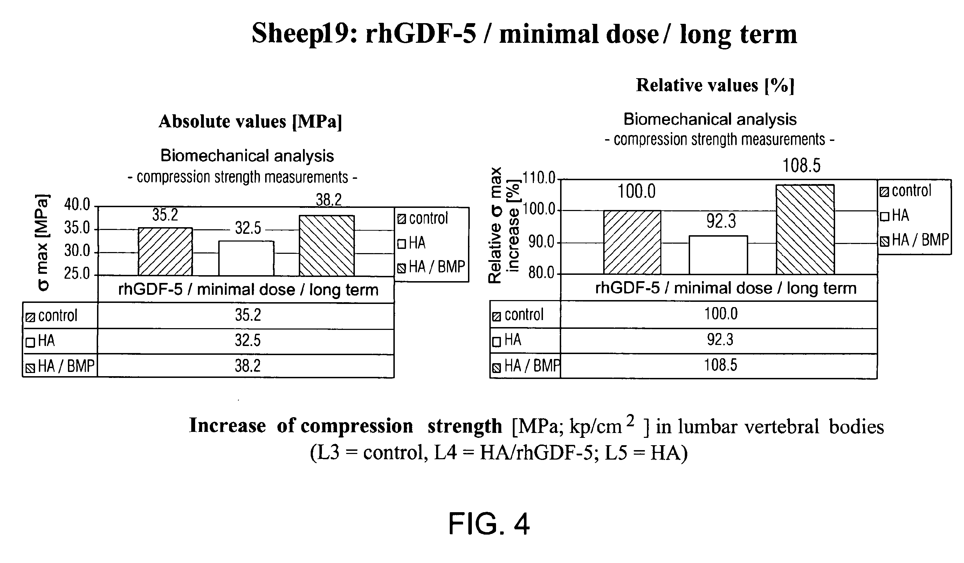

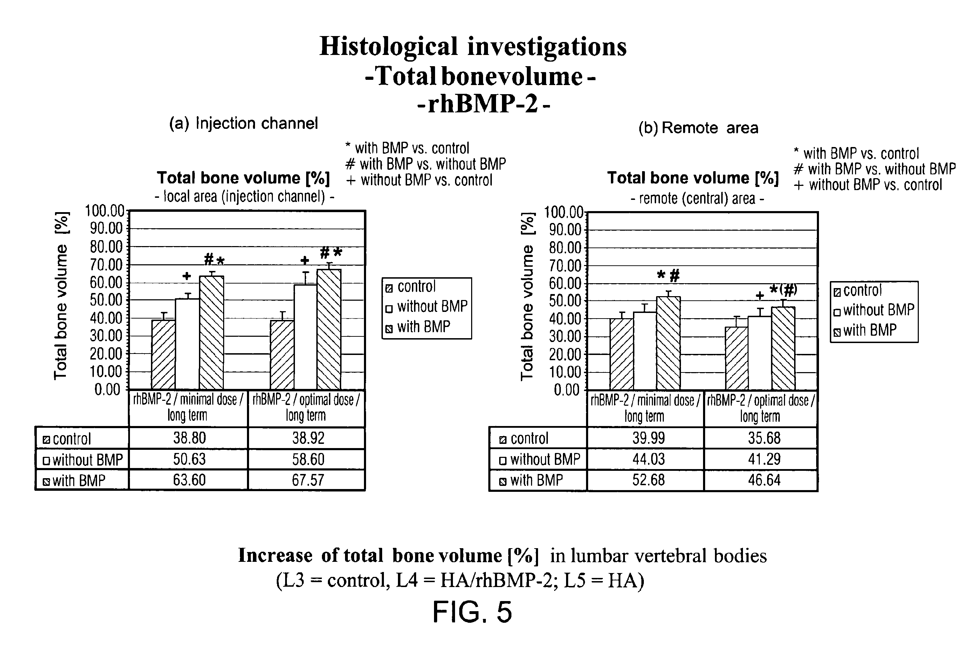

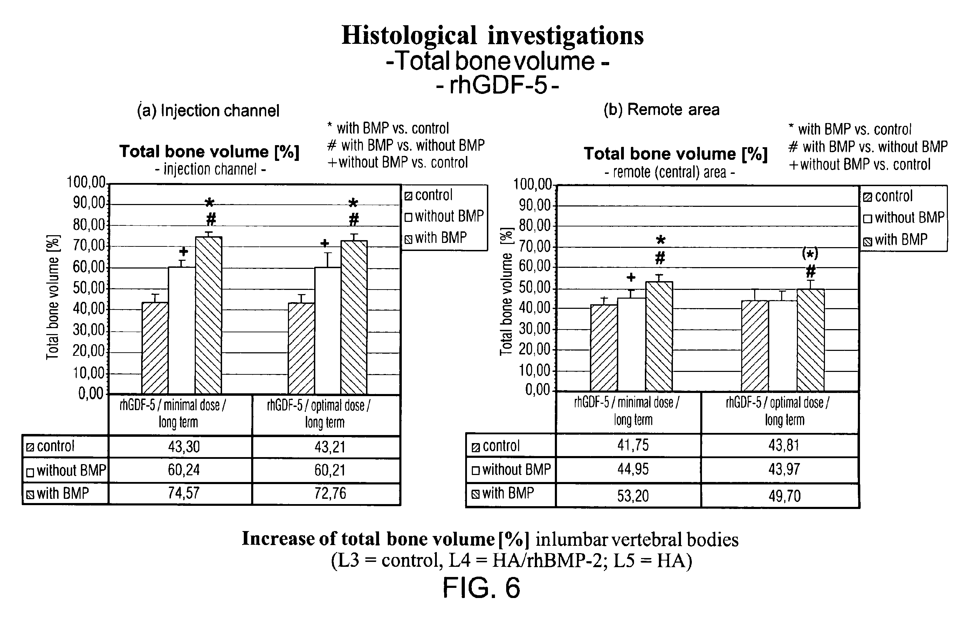

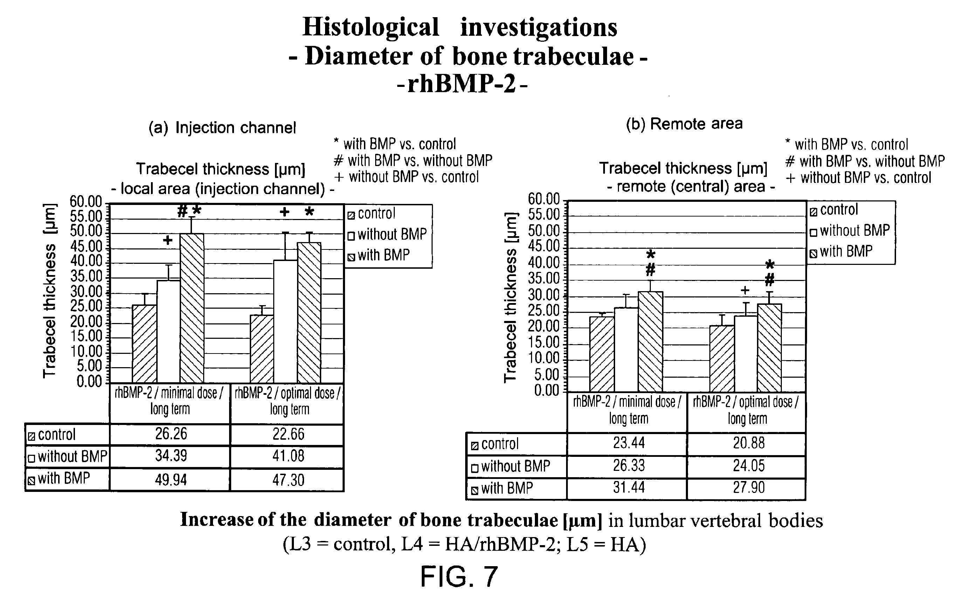

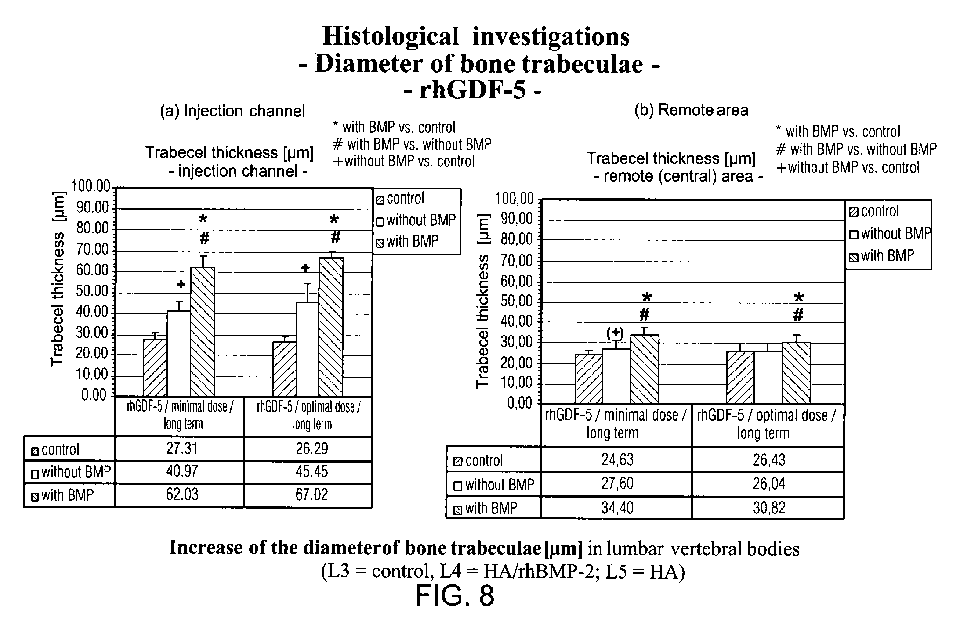

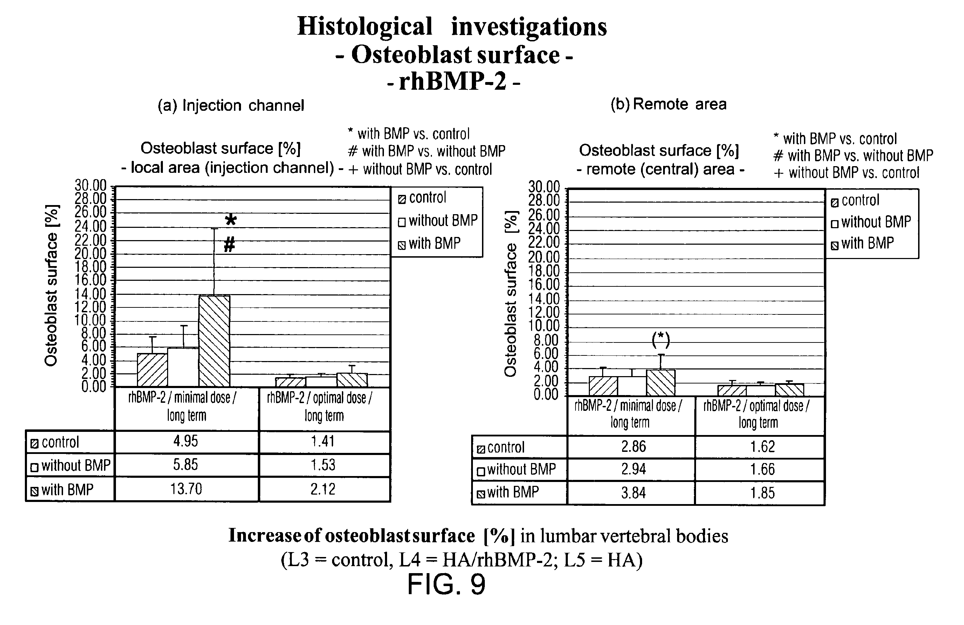

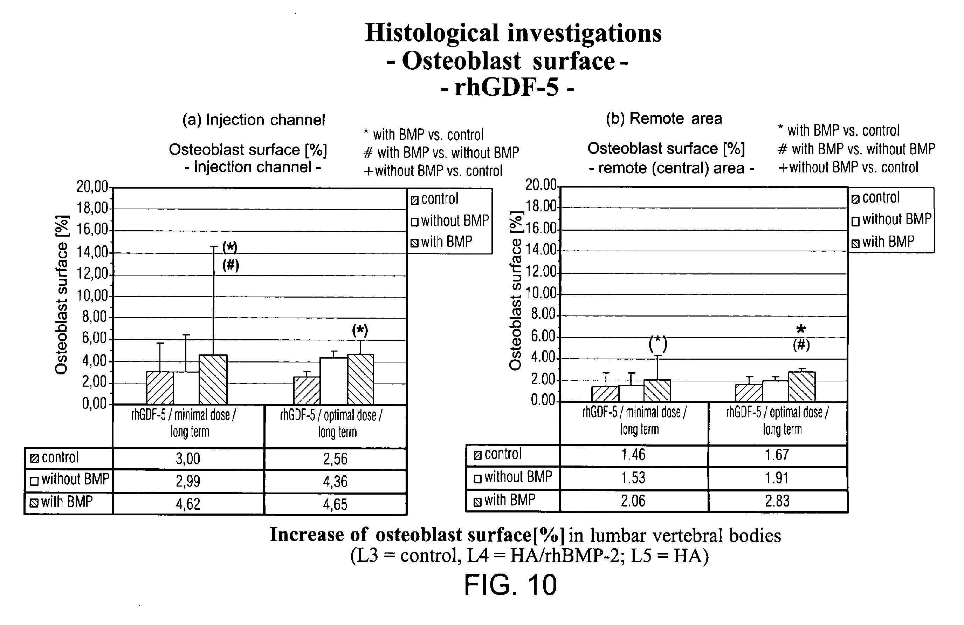

The lumbar vertebral body of the animals was injected with the compositions comprising rh-BMP-2 or rhGDF-5. After three months a significant increase in the bone mineral density (BMD) could be observed and was still present after nine months. The BMD was measured in the central spongious part of the vertebral body excluding the boney cortical area using the DEXA QDR 4500 Elite (HOLOGIC) as a BMD measurement device. The results are shown in Fig. 2. GDF-5, which previous data showed to exhibit only about 20% of the activity of BMP-2 and thus require a five times higher dosages to achieve results equivalent to BMP-2 was found to result in about the same, and even slightly higher bone density, at half the concentration of BMP-2 (10 µg GDF-5 vs. 20 µg BMP-2). Thus, GMP-5 when attached to CaP carriers opens an avenue of providing osteogenic factors at a lower dosage than the currently available BMP-2 and BMP-7 compositions. The study also included measurement of the compression strength in which extracted 10 x 15 mm cylinders from prepared and treated vertebral bodies (control, HA, and "HA-BMP" (HA-BMP-2/ HA-GDF-5) were subjected to compression. Figs. 3 and 4 show that both in absolute terms, as well as in relative terms, the HA together with the "minimal" dose of GDF-5 (10 µg) compares well with the higher, "maximal" dose of BMP-2 (20 µg) in these compression studies. Figs. 5 and 6 also show that the "minimal" dose of GDF-5 (10 µg) compares well with the "maximal" dose of BMP-2 (20 µg) in terms of total bone volume. In both cases, an about 30% increase at the injection channel could be shown as well as an about 10% increase in the remote central area. The remote area can have a distance of more than 5 mm and up to 10 mm from the injection channel, showing that the composition of the present invention displayed activity beyond an area that the person skilled in the art would have expected to be reached by simple diffusion. Figs. 7 and 8 also show that the "minimal" dose of GDF-5 (10 µg) compares well with the "maximal" dose of BMP-2 (20 µg) in terms of their effect on diameter of bone trabeculae. In both cases, an about 30% increase at the injection channel was observed as well as an about 10% increase in the remote central area. Finally, Figs. 9 and 10 show that the "minimal" dose of GDF-5 (10 µg) compares well with the "maximal" dose of BMP-2 (20 µg) in terms of osteoblast surface. In both cases, an increase at the injection channel was observed as well as some increase in the remote central area.

### Example 2:

A CaP-GDP composition comprising calciumhydrogenphophate anhydride, a microscaffold coated with nanocrystalline calciumhydrogenphophate anhydride, GDP-5 at a total concentration of 100 µg is provided. The composition is admixed with a buffer solution to reach a suitable viscosity for injection and is injected into a fractured vertebral body of a patient with a 9 gauge injection needle having an inner diameter of 2.2 mm. The injected CaP-GDP composition hardens *in situ*. During the hardening process pores are formed. The pores formed have an average diameter of less than 100 µm. The compression strength of the hardened CaP-GDP composition is less than 100 MPa and has an elastic modulus of more than 1000 MPa. GDF is released into the fractured vertebral body and induces and/or furthers osteogenesis in the vertebral body despite the pathological reduction of osteogenesis in the patient. New bone initially forms in the pores of the hardened composition and stepwise replaces parts of the bioresorbable CaP material. After one year 70% of the CaP material is replaced by newly-formed bone.

### Example 3:

Four to six weeks after the procedure in Example 2 has been completed, a composition as described in Example 2 is injected into vertebral bodies adjacent to the fractured vertebral body of the patient in Example 2. Upon injection, bone formation is also induced and/or furthered in those adjacent vertebral bodies to increase their compression strength. This preventive treatment of the adjacent vertebral bodies reduces the risk of subsequent fractures.

Individual Applicant
--------------------
      Street :
      City :
      State :
      Country :
      PostalCode :
      PhoneNumber :
      FaxNumber :
      EmailAddress :
<110> LastName : Venbrocks
<110> FirstName : Rudolf A.
<110> MiddleInitial :
<110> Suffix :

Individual Applicant
--------------------
      Street :
      City :
      State :
      Country :
      PostalCode :
      PhoneNumber :
      FaxNumber :
      EmailAddress :
<110> LastName : Jandt
<110> FirstName : Klaus D.
<110> MiddleInitial :
<110> Suffix :

Individual Applicant
--------------------
      Street :
      City :
      State :
      Country :
      PostalCode :
      PhoneNumber :
      FaxNumber :
      EmailAddress :
<110> LastName : Kinne
<110> FirstName : Raimund W.
<110> MiddleInitial :
<110> Suffix :

Individual Applicant
--------------------
      Street :
      City :
      State :
      Country :
      PostalCode :
      PhoneNumber :
      FaxNumber :
      EmailAddress :
<110> LastName : Bossert
<110> FirstName : Joerg
<110> MiddleInitial :
<110> Suffix :

Individual Applicant
--------------------
      Street :
      City :
      State :
      Country :
      PostalCode :
      PhoneNumber :

FaxNumber :
EmailAddress :
<110> LastName : Hortschansky
<110> FirstName : Peter
<110> MiddleInitial :
<110> Suffix :

Individual Applicant
--------------------
Street :
City :
State :
Country :
PostalCode :
PhoneNumber :
FaxNumber :
EmailAddress :
<110> LastName : Schmuck
<110> FirstName : Klaus
<110> MiddleInitial :
<110> Suffix :

Application Project
-------------------
<120> Title : CALCIUM PHOSPATE BASED DELIVERY OF GROWTH AND DIFFERENTIATION
FACTORS TO COMPROMISED BONE
<130> AppFileReference : DPG153/0A/EP
<140> CurrentAppNumber :
<141> CurrentFilingDate : ____-__-__

Earlier Applications
--------------------
<150> PriorAppNumber : US 60/970,258
<151> PriorFilingDate : 2007-09-05

Earlier Applications
--------------------
<150> PriorAppNumber : US 11/928,889
<151> PriorFilingDate : 2007-10-30

Sequence
--------
<213> OrganismName : Artificial
<400> PreSequenceString :
MNSMDPEST 9
<212> Type : PRT
<211> Length : 9
SequenceName : 1
SequenceDescription :

Sequence
--------
<213> OrganismName : Artificial
<400> PreSequenceString :
atgaaaatcg aagaaggtaa actggtaatc tggattaacg gcgataaagg ctataacggt 60
ctcgctgaag tcggtaagaa attcgagaaa gataccggaa ttaaagtcac cgttgagcat 120
ccggataaac tggaagagaa attcccacag gttgcggcaa ctggcgatgg ccctgacatt 180
atcttctggg cacacgaccg ctttggtggc tacgctcaat ctggcctgtt ggctgaaatc 240
accccggaca aagcgttcca ggacaagctg tatccgttta cctgggatgc cgtacgttac 300
aacggcaagc tgattgctta cccgatcgct gttgaagcgt tatcgctgat ttataacaaa 360
gatctgctgc cgaacccgcc aaaaacctgg gaagagatcc cggcgctgga taaagaactg 420
aaagcgaaag gtaagagcgc gctgatgttc aacctgcaag aaccgtactt cacctggccg 480
ctgattgctg ctgacggggg ttatgcgttc aagtatgaaa acggcaagta cgacattaaa 540
gacgtgggcg tggataacgc tggcgcgaaa gcgggtctga ccttcctggt tgacctgatt 600
aaaaacaaac acatgaatgc agacaccgat tactccatcg cagaagctgc ctttaataaa 660
ggcgaaacag cgatgaccat caacggcccg tgggcatggt ccaacatcga caccagcaaa 720
gtgaattatg gtgtaacggt actgccgacc ttcaagggtc aaccatccaa accgttcgtt 780
ggcgtgctga gcgcaggtat taacgccgcc agtccgaaca aagagctggc aaaagagttc 840

ctcgaaaact atctgctgac tgatgaaggt ctggaagcgg ttaataaaga caaaccgctg 900
ggtgccgtag cgctgaagtc ttacgaggaa gagttggcga aagatccacg tattgccgcc 960
accatggaaa acgcccagaa aggtgaaatc atgccgaaca tcccgcagat gtccgctttc 1020
tggtatgccg tgcgtactgc ggtgatcaac gccgccagcg gtcgtcagac tgtcgatgaa 1080
gccctgaaag acgcgcagac taattcgagc tcgaacaaca acaacaataa caataacaac 1140
aacctcgggc atcaccatca ccatcatgga tccgagaacc tgtacttcca gggtgcccca 1200
ctggccactc gccagggcaa gcgacccagc aagaacctta aggctcgctg cagtcggaag 1260
gcactgcatg tcaacttcaa ggacatgggc tgggacgact ggatcatcgc accccttgag 1320
tacgaggctt tccactgcga ggggctgtgc gagttcccat tgcgctccca cctggagccc 1380
acgaatcatg cagtcatcca gaccctgatg aactccatgg accccgagtc cacaccaccc 1440
acctgctgtg tgcccacgcg gctgagtccc atcagcatcc tcttcattga ctctgccaac 1500
aacgtggtgt ataagcagta tgaggacatg gtcgtggagt cgtgtggctg caggtag 1557

<212> Type : DNA
<211> Length : 1557
SequenceName : 2
SequenceDescription :

Sequence
--------
<213> OrganismName : Artificial
<400> PreSequenceString :
MKIEEGKLVIWINGDKGYNGLAEVGKKFEKDTGIKVTVEHPDKLEEKFPQVAATGDGPDIIFWAHDRFGGYAQSGLLAEI
TPDKAFQDKLYPFTWDAVRYNGKLIAYPIAVEALSLIYNKDLLPNPPKTWEEIPALDKELKAKGKSALMFNLQEPYFTWP
LIAADGGYAFKYENGKYDIKDVGVDNAGAKAGLTFLVDLIKNKHMNADTDYSIAEAAFNKGETAMTINGPWAWSNIDTSK
VNYGVTVLPTFKGQPSKPFVGVLSAGINAASPNKELAKEFLENYLLTDEGLEAVNKDKPLGAVALKSYEEELAKDPRIAA
TMENAQKGEIMPNIPQMSAFWYAVRTAVINAASGRQTVDEALKDAQTNSSSNNNNNNNNNNLGHHHHHHGSENLYFQGAP
LATRQGKRPSKNLKARCSRKALHVNFKDMGWDDWIIAPLEYEAFHCEGLCEFPLRSHLEPTNHAVIQTLMNSMDPESTPP
TCCVPTRLSPISILFIDSANNVVYKQYEDMVVESCGCR
<212> Type : PRT
<211> Length : 518
SequenceName : 3
SequenceDescription :

Sequence
--------
<213> OrganismName : Homo sapiens
<400> PreSequenceString :
GAPLATRQGK RPSKNLKARC SRKALHVNFK DMGWDDWIIA PLEYEAFHCE GLCEFPLRSH 60
LEPTNHAVIQ TLMNSMDPES TPPTCCVPTR LSPISILFID SANNVVYKQY EDMVVESCGC 120
R 121
<212> Type : PRT
<211> Length : 121
SequenceName : 4
SequenceDescription :

SEQUENCE LISTING

<110> Venbrocks, Rudolf A.
      Jandt, Klaus D.
      Kinne, Raimund W.
      Bossert, Joerg
      Hortschansky, Peter
      Schmuck, Klaus

<120> CALCIUM PHOSPATE BASED DELIVERY OF GROWTH AND DIFFERENTIATION
      FACTORS TO COMPROMISED BONE

<130> DPG153/0A/EP

<150> US 60/970,258
<151> 2007-09-05

<150> US 11/928,889
<151> 2007-10-30

<160> 4

<170> PatentIn version 3.3

<210> 1
<211> 9
<212> PRT
<213> Artificial

<220>
<223> Partial GDF-5 sequence

<400> 1

Met Asn Ser Met Asp Pro Glu Ser Thr
1               5


<210> 2
<211> 1557
<212> DNA
<213> Artificial

<220>
<223> Coding sequence for fusion protein of maltose-binding protein
      (MBP) and GDF-5

<400> 2
atgaaaatcg aagaaggtaa actggtaatc tggattaacg gcgataaagg ctataacggt      60

ctcgctgaag tcggtaagaa attcgagaaa gataccggaa ttaaagtcac cgttgagcat     120

ccggataaac tggaagagaa attcccacag gttgcggcaa ctggcgatgg ccctgacatt     180

atcttctggg cacacgaccg ctttggtggc tacgctcaat ctggcctgtt ggctgaaatc     240

accccggaca aagcgttcca ggacaagctg tatccgttta cctgggatgc cgtacgttac     300

aacggcaagc tgattgctta cccgatcgct gttgaagcgt tatcgctgat ttataacaaa     360

gatctgctgc cgaacccgcc aaaaacctgg gaagagatcc cggcgctgga taaagaactg     420

aaagcgaaag gtaagagcgc gctgatgttc aacctgcaag aaccgtactt cacctggccg     480

ctgattgctg ctgacggggg ttatgcgttc aagtatgaaa acggcaagta cgacattaaa     540

gacgtgggcg tggataacgc tggcgcgaaa gcgggtctga ccttcctggt tgacctgatt     600

aaaaacaaac acatgaatgc agacaccgat tactccatcg cagaagctgc ctttaataaa 660

ggcgaaacag cgatgaccat caacggcccg tgggcatggt ccaacatcga caccagcaaa 720

gtgaattatg gtgtaacggt actgccgacc ttcaagggtc aaccatccaa accgttcgtt 780

ggcgtgctga gcgcaggtat taacgccgcc agtccgaaca aagagctggc aaaagagttc 840

ctcgaaaact atctgctgac tgatgaaggt ctggaagcgg ttaataaaga caaaccgctg 900

ggtgccgtag cgctgaagtc ttacgaggaa gagttggcga aagatccacg tattgccgcc 960

accatggaaa acgcccagaa aggtgaaatc atgccgaaca tcccgcagat gtccgctttc 1020

tggtatgccg tgcgtactgc ggtgatcaac gccgccagcg gtcgtcagac tgtcgatgaa 1080

gccctgaaag acgcgcagac taattcgagc tcgaacaaca acaacaataa caataacaac 1140

aacctcgggc atcaccatca ccatcatgga tccgagaacc tgtacttcca gggtgcccca 1200

ctggccactc gccagggcaa gcgacccagc aagaacctta aggctcgctg cagtcggaag 1260

gcactgcatg tcaacttcaa ggacatgggc tgggacgact ggatcatcgc accccttgag 1320

tacgaggctt tccactgcga ggggctgtgc gagttcccat tgcgctccca cctggagccc 1380

acgaatcatg cagtcatcca gaccctgatg aactccatgg accccgagtc cacaccaccc 1440

acctgctgtg tgcccacgcg gctgagtccc atcagcatcc tcttcattga ctctgccaac 1500

aacgtggtgt ataagcagta tgaggacatg gtcgtggagt cgtgtggctg caggtag 1557

<210> 3
<211> 518
<212> PRT
<213> Artificial

<220>
<223> Fusion protein: Maltose-binding protein (MBP) and GDF-5

<400> 3

Met Lys Ile Glu Glu Gly Lys Leu Val Ile Trp Ile Asn Gly Asp Lys
1               5                   10                  15

Gly Tyr Asn Gly Leu Ala Glu Val Gly Lys Lys Phe Glu Lys Asp Thr
            20                  25                  30

Gly Ile Lys Val Thr Val Glu His Pro Asp Lys Leu Glu Glu Lys Phe
        35                  40                  45

Pro Gln Val Ala Ala Thr Gly Asp Gly Pro Asp Ile Ile Phe Trp Ala
    50                  55                  60

His Asp Arg Phe Gly Gly Tyr Ala Gln Ser Gly Leu Leu Ala Glu Ile
65                  70                  75                  80

Thr Pro Asp Lys Ala Phe Gln Asp Lys Leu Tyr Pro Phe Thr Trp Asp
                85                  90                  95

Ala Val Arg Tyr Asn Gly Lys Leu Ile Ala Tyr Pro Ile Ala Val Glu

            100                 105                 110

Ala Leu Ser Leu Ile Tyr Asn Lys Asp Leu Leu Pro Asn Pro Pro Lys
        115                 120                 125

Thr Trp Glu Glu Ile Pro Ala Leu Asp Lys Glu Leu Lys Ala Lys Gly
    130                 135                 140

Lys Ser Ala Leu Met Phe Asn Leu Gln Glu Pro Tyr Phe Thr Trp Pro
145                 150                 155                 160

Leu Ile Ala Ala Asp Gly Gly Tyr Ala Phe Lys Tyr Glu Asn Gly Lys
                165                 170                 175

Tyr Asp Ile Lys Asp Val Gly Val Asp Asn Ala Gly Ala Lys Ala Gly
            180                 185                 190

Leu Thr Phe Leu Val Asp Leu Ile Lys Asn Lys His Met Asn Ala Asp
        195                 200                 205

Thr Asp Tyr Ser Ile Ala Glu Ala Ala Phe Asn Lys Gly Glu Thr Ala
    210                 215                 220

Met Thr Ile Asn Gly Pro Trp Ala Trp Ser Asn Ile Asp Thr Ser Lys
225                 230                 235                 240

Val Asn Tyr Gly Val Thr Val Leu Pro Thr Phe Lys Gly Gln Pro Ser
                245                 250                 255

Lys Pro Phe Val Gly Val Leu Ser Ala Gly Ile Asn Ala Ala Ser Pro
            260                 265                 270

Asn Lys Glu Leu Ala Lys Glu Phe Leu Glu Asn Tyr Leu Leu Thr Asp
        275                 280                 285

Glu Gly Leu Glu Ala Val Asn Lys Asp Lys Pro Leu Gly Ala Val Ala
    290                 295                 300

Leu Lys Ser Tyr Glu Glu Glu Leu Ala Lys Asp Pro Arg Ile Ala Ala
305                 310                 315                 320

Thr Met Glu Asn Ala Gln Lys Gly Glu Ile Met Pro Asn Ile Pro Gln
                325                 330                 335

Met Ser Ala Phe Trp Tyr Ala Val Arg Thr Ala Val Ile Asn Ala Ala
            340                 345                 350

Ser Gly Arg Gln Thr Val Asp Glu Ala Leu Lys Asp Ala Gln Thr Asn
        355                 360                 365

Ser Ser Ser Asn Asn Asn Asn Asn Asn Asn Asn Asn Asn Leu Gly His

370                     375                     380

His His His His His Gly Ser Glu Asn Leu Tyr Phe Gln Gly Ala Pro
385                 390                 395                 400

Leu Ala Thr Arg Gln Gly Lys Arg Pro Ser Lys Asn Leu Lys Ala Arg
                405                 410                 415

Cys Ser Arg Lys Ala Leu His Val Asn Phe Lys Asp Met Gly Trp Asp
            420                 425                 430

Asp Trp Ile Ile Ala Pro Leu Glu Tyr Glu Ala Phe His Cys Glu Gly
        435                 440                 445

Leu Cys Glu Phe Pro Leu Arg Ser His Leu Glu Pro Thr Asn His Ala
    450                 455                 460

Val Ile Gln Thr Leu Met Asn Ser Met Asp Pro Glu Ser Thr Pro Pro
465                 470                 475                 480

Thr Cys Cys Val Pro Thr Arg Leu Ser Pro Ile Ser Ile Leu Phe Ile
                485                 490                 495

Asp Ser Ala Asn Asn Val Val Tyr Lys Gln Tyr Glu Asp Met Val Val
            500                 505                 510

Glu Ser Cys Gly Cys Arg
        515

<210>  4
<211>  121
<212>  PRT
<213>  Homo sapiens

<400>  4

Gly Ala Pro Leu Ala Thr Arg Gln Gly Lys Arg Pro Ser Lys Asn Leu
1               5                   10                  15

Lys Ala Arg Cys Ser Arg Lys Ala Leu His Val Asn Phe Lys Asp Met
            20                  25                  30

Gly Trp Asp Asp Trp Ile Ile Ala Pro Leu Glu Tyr Glu Ala Phe His
        35                  40                  45

Cys Glu Gly Leu Cys Glu Phe Pro Leu Arg Ser His Leu Glu Pro Thr
    50                  55                  60

Asn His Ala Val Ile Gln Thr Leu Met Asn Ser Met Asp Pro Glu Ser
65                  70                  75                  80

Thr Pro Pro Thr Cys Cys Val Pro Thr Arg Leu Ser Pro Ile Ser Ile
                85                  90                  95

Leu Phe Ile Asp Ser Ala Asn Asn Val Val Tyr Lys Gln Ty[?] Glu Asp
            100                 105                 11[?]

Met Val Val Glu Ser Cys Gly Cys Arg
        115                 120

## Claims

1. A composition for delivering recombinant growth and differentiation factor(s) comprising:
a bioresorbable calcium phosphate material; and
a recombinant GDF-5 and/or GDF-3 as a biologically active agent,

2. The composition of claim 1, wherein said composition is cell-free.

3. The composition of claim 1 or 2, wherein the recombinant GDF-5 is a non-glycosylated human recombinant GDF-5.

4. The composition according to one of claims 1 to 3 comprising at least one further osteogenic and/or osteoinductive protein selected from bone morphogenic proteins and/or further GDFs and/or a transforming growth factors.

5. The composition according to one of claims 1 to 4, wherein said recombinant GDF-5 comprises SEQ ID NO: 1: MNSMDPEST.

6. The composition according to one of claims 1 to 5, wherein said composition is injectable, preferably in form of a solution or paste or putty.

7. The composition according to one of claims 1 to 6, wherein the calcium phosphate material is selected from the group of calcium phosphates consisting of monocalciumphosphate anhydrite, calciumhydrogenphosphate dihydrate, calciumhydrogenphosphate anhydrite, hydroxyapatite, a-tricalcium phosphate, ß-tricalcium phosphate, fluorapatite, and a combination thereof.

8. The composition according to one of claims 1 to 7, wherein the calcium phosphate material is, upon hardening, strongly bioresorbable.

9. The composition according to one of claims 1 to 8, wherein the calcium phosphate material, upon hardening, comprises phases that resorb at different rates.

10. The composition of claim 9, wherein, upon hardening, a first phase resorbs at first rate and optionally comprises pores having an average diameter of less than one µm and a second phase resorbs at a second rate which exceeds that of said first rate.

11. The composition of claim 10, wherein said pores comprise portions of said second phase and said first phase optionally essentially consists of particles.

12. The composition according to one of claims 1 to 11, further comprising a gas-forming agent.

13. The composition according to one of claims 1 to 12, wherein said composition has, upon hardening, a compression strength of more than about 20 MPa, but less than about 200 MPa.

14. The composition according to one of claims 1 to 13, wherein said calcium phosphate material comprises, upon hardening, interconnected pores.

15. The composition according to one of claims 1 to 14, wherein said composition further comprises a bioerodible polymeric structure.

16. The composition according to claim 15, wherein said polymeric structure is a polysaccharide, peptide, protein, synthetic polymer, natural polymer, a synthetic or natural copolymer, or combinations thereof.

17. The composition according to claim 15 or 16, wherein said polymeric structure is/are microscaffold(s) having a porous structure.

18. The composition according to claim 17, wherein said microscaffold(s) is/are between 500 µm and 1 mm in size and optionally is/are in the form of hollow beads.

19. The composition according to claim 17 or 18, wherein said microscaffold(s) has/have pore channel diameters of about 30 to 500 µm, preferably of about 100 to 200 µm.

20. The composition according to one of claims 17 to19, wherein said microscaffold(s) has/have a diameter that exceeds the inner diameter of a syringe with which the composition is injected.

21. The composition according to one of claims 17 to 20, wherein said microscaffold is coated with a biocompatible nanocrystalline calcium phosphate.

22. A composition according to claim 21, wherein said nanocrystalline calcium phosphate is selected from the group consisting of monocalciumphosphate anhydrite, calciumhydrogenphosphate dihydrate, calciumhydrogenphosphate anhydrite, hydroxyapatite, a-tricalcium phosphate, β-tricalcium phosphate, fluorapatite, and a combination thereof.

23. A kit comprising:
(a) the bioresorbable calcium phosphate material of one of claims 1 or 6 to 13 in one container;
(b) the biologically active agent of one of claims 1, 2 or 4 in the same or a separate container; and
(c) in a further container, instructions of how to use (a) and (b).

24. The kit of claim 23, wherein the biologically active agent is admixed in a buffer solution or with the bioresorbable calcium phosphate material.

25. The kit of claim 23 or 24, wherein the kit further comprises tools for kyphoplasty or vertebroplasty, or a porous pouch.

26. The kit of claim 25, wherein said pouch is made of a polyester material, preferably PET.

27. The kit of claim 25 or 26, wherein pores of said pouch have a diameter of less than about 1 mm.

28. A method for preparing a porous, bioresorbable, GDF releasing calcium phosphate cement comprising:
- providing a combination of a bioresorbable calcium phosphate material and a GDF, preferably GDF-5 and/or GDF-3 as a biologically active agent; and
- mixing said combination so that pores are formed upon hardening to produce a porous, bioresorbable, GDF releasing calcium phosphate cement.

29. The method of claim 28, wherein said pores are elongated and have a diameter of about 100 µm to about 1 mm, preferably 100 µm to about 200 µm.

30. The method of claim 28 or 29, wherein said pores have a length of more than 1 mm.

31. The method of claim 28, wherein said pores are spherical pores with a diameter of less than 1 µm.

32. The method according to one of claims 28 to 31, wherein said calcium phosphate cement comprises elongated and spherical pores.

33. Use of a composition according to one of claims 1 to 22, for the manufacture of a medicament.

34. The use of claim 33, wherein the medicament is a medicament for furthering bone formation, preferably in a mammalian organism:

35. The use of claim 33 or 34, wherein the medicament is suitable for the treatment of osteoporosis and other diseases associated with bone degeneration and/or bone fractures.

36. The use according to one of claims 33 to 35, wherein said medicament is for administering in one or more doses of less than 100 µg.

37. The use according to one of claims 33 to 36, wherein said medicament is for administering according to an administration pattern comprising measuring the bone mineral density (BMD) of a bone comprising said bone location and determining a lower threshold value of said BMD.

38. The use according to one of claims 33 to 37, wherein said medicament is for administering according to an administration pattern comprising providing a cavity at said bone location, inserting a porous pouch into said cavity and administering said composition into said porous pouch.

39. The use according to claim 38, wherein said medicament is for administering via a single passage to said cavity.

40. The use according to one of claims 33 to 39, wherein administering of said medicament results in an at least about 20% increase in bone volume at said bone location.
